# DEMANDE DE BREVET EUROPEEN

(11) **EP 1 602 368 A2**
(43) Date de publication de la demande: **07.12.2005**
(21) Numéro de dépôt: 05018614.7
(22) Date de dépôt: 26.07.2001
(51) Int. Cl.: A61K 31/137, A61K 31/495, A61K 31/381, A61P 43/00, A61P 17/14, A61P 35/00, A61P 33/00, A61P 31/12, A61P 25/28, A61P 21/00, A61K 31/166, A61K 31/222, A61K 31/661, A61K 31/18, A61K 31/4164, A61K 31/63, C07C 211/53, C07D 295/155

(54) **Inhibiteurs de phosphatases cdc25**

(30) Priorité: 28.07.2000 FR 0009900
(62) Demande divisionnaire de: 01960837.1
(71) Demandeur: Société de Conseils de Recherches et d'Applications Scientifiques ( S.C.R.A.S.), 75016 Paris (FR)
(72) Inventeur: Prevost, Grégoire, 92160 Antony (FR); Brezak Pannetier, Marie-Christine, 92160 Antony (FR); Galcera Contour, Marie-Odile, 91070 Bondoufle (FR); Thurieau, Christophe, 75116 Paris (FR); Goubin Gramatica, Francoise, 47000 Agen (FR); Ducommun, Bernard, 31450 Belberaud (FR); Lanco, Christophe, 91410 Dourdan (FR)
(74) Mandataire: Bourgouin, André

(57) **Abrégé**

L'invention a pour objet de nouveaux inhibiteurs de phosphatases cdc25, et en particulier de phosphatase cdc25-C, lesquels répondent à la formule générale (I) 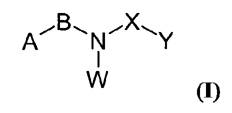 dans laquelle :
A représente un radical aryle carbocyclique éventuellement substitué de 1 à 3 fois par un ou des radicaux choisis indépendamment parmi un atome halogène et un radical alkyle, hydroxy, alkoxy, alkylthio ou NR¹R² dans lequel R¹ et R² représentent un atome d'hydrogène ou un radical alkyle ou R¹ et R² forment ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR³R⁴-, -O-, -S- et -NR⁵-, R³ et R⁴ représentant indépendamment à chaque fois qu'ils interviennent un atome d'hydrogène ou un radical alkyle, hydroxy, alkoxy, amino, alkylamino ou dialkylamino, et R⁵ représentant indépendamment à chaque fois qu'il intervient un atome d'hydrogène ou un radical alkyle,
   ou encore A représente un radical phényle substitué par un radical phényle éventuellement substitué de 1 à 3 fois par un ou des radicaux choisis indépendamment parmi un atome halogène ou un radical alkyle, hydroxy, alkoxy, alkylthio ou NR¹R² dans lequel R¹ et R² représentent un atome d'hydrogène ou un radical alkyle ;
B représente un radical -(CH₂)ᵢ-CO-, -NH-CO-(CH₂)ₙ- ou -(CH₂)ₚ-, i et n étant des entiers de 0 à 2 et p étant un entier de 0 à 1 ;
W représente un atome d'hydrogène ou un radical alkyle ;
X représente un radical -(CH₂)_{q}- ou -(CH₂)ⱼ-CO-(CH₂)ᵣ-, q étant un entier de 0 à 4 et j et r étant des entiers de 0 à 6 ;
Y représente notamment un radical nitrophényle, aminophényle, alkylaminophényle ou dialkylaminophényle ou encore le radical (T) représenté ci-dessous

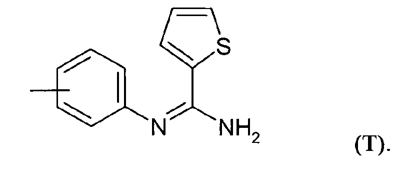

## Description

Le jeu de revendications de la présente demande divisionnaire a été modifié par rapport au jeu de revendications de la demande telle que déposée. Les modifications peuvent être résumées comme suit:
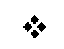 Les revendications 1 à 10 telles que déposées ont été supprimées.
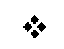 L'ancienne revendication 11 telle que déposée sert de base à la nouvelle revendication 1. L'objet de la nouvelle revendication 1 est limité à des composées de formule (I) ayant un groupement N(W)-X acyclique. Les composées de formule (I) ayant un groupement N(W)-X représentant un hétérocycle à 2 amines ont été supprimés, le reste de la revendication restant inchangé.
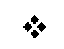 Une nouvelle revendication 2 dépendant de la nouvelle revendication 1 est ajoutée. Les caractéristiques techniques supplémentaires pour cette revendication trouvent leur contrepartie dans la description de la demande telle que déposée (cf. demande telle que déposée, passage page 16, ligne 23 à page 19, ligne 20 pris avec le passage en page 37, lignes 20-22).
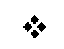 Une nouvelle revendication 3 a été ajoutée. Tous les composés dans lesquels N(W)-X représente un hétérocycle à 2 amines ont été supprimés (c'est-à-dire les composés des exemples 31 à 33 qui sont les 3 derniers composés de la liste de l'ancienne revendication 12) ainsi que les composés pour lesquels Y n'est pas un groupe de type sulfonamide ou de type phosphate.
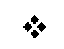 L'ancienne revendication 13 a été supprimée.
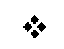 Les anciennes revendications 14 et 15 telles que déposées deviennent respectivement les nouvelles revendications 4 et 5. Dans un souci d'harmonisation des nomenclatures, il est proposé de transformer les noms de toutes les formules générales dérivées de la formule générale **(I)** (par exemple « **(I).3** ») de sorte qu'ils soient dérivés de la formule générale **(III),** sachant que les composés préparés selon le procédé sont des composés particuliers de formule générale **(III)** selon la nouvelle revendication 1.
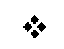 L'ancienne revendication 16 a été supprimée.
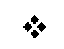 L'ancienne revendication 17 telle que déposée devient la nouvelle revendication 6. Dans un souci d'harmonisation des nomenclatures, il est proposé de transformer les noms de toutes les formules générales dérivées de la formule générale **(I)** (par exemple « **(I).9** ») de sorte qu'ils soient dérivés de la formule générale **(III),** sachant que les composés préparés selon le procédé sont des composés particuliers de formule générale **(III)** selon la nouvelle revendication 1.
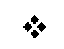 Une nouvelle revendication 7 est ajoutée. Les caractéristiques techniques pour cette revendication trouvent leur contrepartie dans la description de la demande telle que déposée (cf. demande telle que déposée, page 37, lignes 1-22).
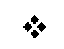 Une nouvelle revendication 8 est ajoutée. Les caractéristiques techniques pour cette revendication trouvent leur contrepartie dans la description de la demande telle que déposée (cf. demande telle que déposée, page 37, lignes 1-3).
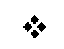 De nouvelles revendications 9 à 11 ont été ajoutées. Les caractéristiques techniques pour cette revendication trouvent leur contrepartie dans la description de la demande telle que déposée (cf. demande telle que déposée, page 37, lignes 4-19).

Les modifications effectuées ne vont pas au-delà du contenu de la demande telle que déposée.

La demanderesse considère donc que les exigences de l'article 123(2) CBE sont de ce fait respectées.

La présente invention a pour objet de nouveaux inhibiteurs de phosphatases cdc25, et en particulier de phosphatase cdc25-C.

Le contrôle de la transition entre les différentes phases du cycle cellulaire durant la mitose ou de la méiose est assurée par un ensemble de protéines dont les activités enzymatiques sont associées à des états différents de phosphorylation. Ces états sont contrôlés par deux grandes classes d'enzymes : les kinases et les phosphatases.

La synchronisation des différentes phases du cycle cellulaire permet ainsi la réorganisation de l'architecture cellulaire à chaque cycle dans l'ensemble du monde vivant (microorganismes, levure, vertébrés, plantes). Parmi les kinases, les kinases dépendantes des cyclines (CDKs) jouent un rôle majeur dans ce contrôle du cycle cellulaire. Leurs activités sont régulées par leurs associations moléculaires avec d'autres protéines appelées cyclines. De plus, des inhibiteurs endogènes sont capables de prévenir ces activités. Plusieurs inhibiteurs de cette famille de kinases sont déjà identifiés et étudiés dans plusieurs domaines thérapeutiques comme l'oncologie pour prévenir la division des cellules tumorales (McDonald et el-Deiry, *Int. J. Oncol*. (2000), 16, 871-886) ou encore la neurobiologie pour prévenir l'apoptose naturelle ou chimio-induite des cellules normales (par exemple les neurones) (cf. Maas et coll., *J. Neurochem.* (1998), **70**, 1401-1410 ; Park et coll., *J. Neurosci.* (1997) **17**, 1256-1270).

Par ailleurs, l'activité enzymatique de ces différentes CDKs est contrôlée par deux autres familles d'enzymes qui travaillent en opposition (Jessus et Ozon, *Prog. Cell Cycle Res.* (1995), **1,** 215-228). La première regroupe des kinases telles que Wee1 et Mik1 qui désactivent les CDKs en phosphorylant certains acides aminés (Den Haese et coll., *Mol. Biol. Cell* (1995), **6,** 371-385). La seconde regroupe des phosphatases telle que Cdc25 qui activent les CDKs en déphosphorylant des résidus tyrosine et thréonine de CDKs (Gould et coll., *Science* (1990), **250,** 1573-1576). La déphosphorylation se ferait grâce à une interaction protéine/protéine entre la cycline et cdc25 dans un premier temps, et ce complexe irait cibler CDK dans un second temps (Morris et Divita, *J. Mol. Biol.* (1999), **286,** 475-487). De plus, la cycline B est elle même phosphorylée par la kinase Cdc2 (cdk1) à laquelle elle est associée (Borgne et coll., *J. Biol. Chem.* (1999), **274,** 11977-11986).

Si une seule forme de cdc25 est décrite dans la levure, une famille de 3 gènes, cdc25-A, cdc25-B et cdc25-C, codent pour les protéines cdc25 humaines. De plus, des variants issus de splicing alternatif du gène cdc25B ont été identifiés : il s'agit de cdc25B1, cdc25B2 et cdc25B3 (Baldin et coll., *Oncogene* (1997), **14 ,** 2485-2495). Les protéines codées par ces variants seraient localisées différemment dans la cellule (Davezac et coll., *Oncogene* (2000), **19,** 2179-2185). L'activité de cdc25 est régulée par les kinases Cdc2 et Cdk2. Mais en absence de la kinase cdc2, l'activité de cdc25 peut être activée par d'autres kinases (Izumi et Maller, *Mol*. *Biol*. *Cell* (1995), **6,** 215-226). Parmi celles-ci, la protéine chk1 phosphoryle cdc25-C sur une sérine en position 216, ce qui augmente son affinité pour une protéine chaperonne 14-3-3. Cette liaison neutralise cdc25-C et maintient en conséquence l'enzyme cdk1 sous un état phosphorylé et donc inactif, ne permettant pas une entrée en mitose. La protéine chaperone permet au complexe de passer dans le cytoplasme grâce un motif protéique d'exportation du noyau (Lopez-Girona et coll., *Nature* (1999), **397**, 172-175).

Un inhibiteur chimique de chk1 (SB-218070) permet à une cellule de poursuivre son cycle cellulaire malgré l'induction de cassure de l'ADN. Cet aspect permet d'augmenter l'efficacité de certains composés cytotoxiques comme la campthotécine (Jackson et coll., *Cancer Res.* (2000), **60,** 566-572).

Le rôle des phosphatases cdc25 dans l'oncogénèse a été décrit initialement par le groupe de Beach montrant que cdc25A et cdc25B en coopérant avec Ha-RASG12V forment des foci après transfection des cellules normales (Galaktionov et coll., *Science* (1995), **269**, 1575-1577). L'activité transformante de cdc25A et cdc25B est observée aussi quand la transfection est faîte dans des cellules ayant une perte du gène suppresseur de tumeur RB1. De plus, l'expression des gènes de cdc25-A et -B semble être sous le contrôle direct de la protéine codée par l'oncogène c-Myc (Galaktionov et coll., *Nature* (1996), **382,** 511-517). Par contre, la phosphatase cdc25-C ne semble pas être contrôlée par cette dernière.

La surexpression de cdc25, et principalement de cdc25-A, semble empêcher la cellule d'arrêter son cycle cellulaire en cas d'agression sur le génome et ainsi éviter un éventuel processus de réparation (Mailand et coll., *Science* (2000), **288**, 1425-1429).

Par ailleurs, la surexpression des différentes formes de cdc25 est maintenant reportée dans de nombreuses séries de tumeurs humaines :
- *Cancer du sein* : la mesure par riboprobe montre que 32% des tumeurs sur-expriment cdc25-B. La surexpression de cdc25-A est montrée dans près de 50% des cancers du sein et est associée à un mauvais pronostic (Cangi et coll., *Résumé 2984, AACR meeting San Francisco,* 2000).
- *Lymphomes* : dans les lymphocytes circulants, les expressions des ARNs de cdc25-B1 et -B3 sont détectées par RT-PCR alors que les expressions de cdc25-A, -B2 et -C sont très faibles ou non détectables. Par contre, l'analyse des ces gènes dans les lymphomes non hodgkiniens montre une forte expression de cdc25-A et -B2 dans environ 35% des tumeurs. Les variants cdc25-B1 et -B3 sont eux détectés dans toutes les tumeurs analysées. Par contre, l'expression de cdc25-C reste très faible dans l'ensemble des prélevements (Hemandez et coll., *Int. J. Cancer* (2000), **89,** 148-152). Il est important de noter la corrélation entre l'expression de protéines telles que myc et cdc25. 26 des 35 (74%) lymphomes non hodgkiniens avec un taux élevé de cdc25-B montre aussi une sur-expression de c-myc. Par contre, 27 des 28 (96%) des tumeurs avec un faible taux de cdc25-B expression ne montrent pas de c-myc détectable (P < 0,0001). Ceci suggère que l'expression de cdc25 associée à celle de myc pourrait participer au développement de ce type de lymphomes (Hernandez et coll., *Cancer Res.* (1998), **58,** 1762-1767).
- *Cancers du cou et de la tête :* sur 20 tumeurs examinées par RT-PCR quantitative, Gasparotto et coll notent que CDC25-A et -B sont surexprimés alors que cdc25-C est très peu exprimé (Gasparotto et coll., *Cancer Res.* (1997), **57,** 2366-2368).

Par ailleurs, le groupe de E. Sausville rapporte une corrélation inverse entre le niveau d'expression de cdc25-B dans un panel de 60 lignées et leurs sensibilités aux inhibiteurs de CDK, comme l'Olomucine ou le Flavopiridol suggérant que la présence de cdc25 puisse apporter une résistance à certains agents antitumoraux et plus particulièrement aux inhibiteurs de CDK (Hose et coll., *Proceedings of AACR,* Abstract 3571, San Francisco, 2000).

La vitamine K3, nommée aussi ménadione, fut le premier inhibiteur sélectif de phosphatase cdc25 décrit (Ham et coll., *Bioorg. Med. Chem. Lett.* (1998) 8, 2507-2510). Depuis, d'autres inhibiteurs de cdc25 ont été identifiés et possèdent une activité inhibitrice de l'ordre du micromolaire sur les enzymes recombinantes. Parmi ces produits, on peut noter :
1. Les analogues de naphtoquinones dérivés de la ménadione (Ham et coll., *Bioorg. Med. Chem. Lett*. (1998) **8**, 2507-2510).
2. Cpd5, un dérivé thioalkyle de la vitamine K (Tamura et coll., *Cancer Res.* (2000), **60,** 1317-1325). Les constantes d'inhibition (Kis) mesurée sur cdc25-A, -B2 & -C sont 15, 1,7 et 1,3 µmol respectivement.
3. L'acide 4-(benzyl-(2-[(2,5-diphényl-oxazole-4-carbonyl)-amino]-éthyl)-carbamoyl)-2-décanoylaminobutanoïque aussi appelé SC-alpha alpha delta 9 (Tamura et coll., *Oncogene* (1999) **18,** 6989-6996).
4. Certains des composés issus d'une librairie de Ugi contenant des groupements inimant les phosphates sont des inhibiteurs non compétitifs de cdc25-A qui n'agissent pas sur le site actif. Le composé le plus actif possède une CI₅₀ de 0,5 µM et le site d'interaction est en cours d'identification (Bergnes et coll., *Bioorg. Med. Chem. Lett*. (1999), **9**, 2849-2854).
5. Les dérivés de la quinolin-4-one et de la 1,7-naphthyridin-4-one. Certains de ces composés sont à la fois inhibiteurs de cdc25 mais aussi de cdc2 (el-Subbagh et coll., *Arch. Pharm. (Weinheim.)* (1999), **332**, 19-24).
6. Le dysidiolide et dérivés contenant un groupe γ-hydroxy buténolide. L'efficacité de ces produits est discutée dans Blanchard et coll., *Bioorg. Med. Chem. Lett.* (1999), **9**, 2537-2538.
7. Certaines 2-bromoindolo[3,2-b]quinoxalines 5-substituées. Certains de ces composés sont à la fois inhibiteurs de cdc25 mais aussi de cdc2 (Abadi et coll., *Arch. Pharm. (Weinheim.)* (1998), **331**, 352-358).

La demande PCT WO 00/17190 décrit des dérivés d'amidines qui inhibent les NO synthases et piègent les radicaux libres. Ces composés présentent de ce fait de nombreuses propriétés pharmacologiques et leur utilisation peut être envisagée dans le traitement de nombreuses pathologies, principalement dans le domaine de la neurologie. Une formule générale simplifiée de ces composés pourrait être la formule générale (ET1): 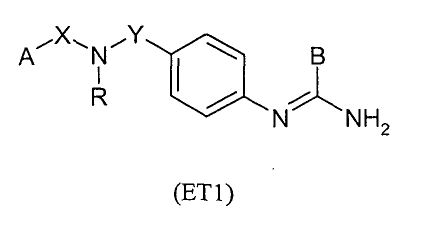 dans laquelle
A représente un radical piégeur de radicaux libres, par exemple un radical phényle substitué ;
X et Y sont des chaînes de liaison, par exemple des radicaux alkylène, alkylènecarbonyle, carbonylalkylène ;
R représente H ou alkyle ; et
B représente un radical aryle carbocyclique ou hétérocyclique, et de préférence le radical 2-thiényle.

L'invention offre de nouveaux inhibiteurs de cdc25, et en particulier de cdc25-C, lesquels répondent à la formule générale **(I)** définie ci-après. Ces composés sont susceptibles d'être utilisés comme médicaments, en particulier dans le traitement des maladies / désordres suivants :
- l'inhibition de la prolifération tumorale seule ou en combinaison avec d'autres traitements ;
- l'inhibition de la prolifération des cellules normales seule ou en combinaison avec d'autres traitements ;
- la prévention de l'alopécie spontanée ;
- la prévention de l'alopécie induite par des produits exogènes ;
- la prévention de l'alopécie radio-induite ;
- la prévention de l'apoptose spontanée ou induite des cellules normales ;
- la prévention de la méiose et la fécondation ;
- la prévention de la maturation des oocytes ;
- toutes les maladies / tous les désordres correspondant à des utilisations rapportées pour les inhibiteurs de CDKs, et notamment les maladies prolifératives non tumorales (par exemple : angiogénèse, psoriasis ou resténose), maladies prolifératives tumorales, parasitologie (prolifération de protozoaires), infections virales, maladies neurodégénératives, myopathies ;
- toutes les maladies / tous les désordres correspondant à des applications cliniques de la vitamine K et de ses dérivés.

Par ailleurs, les composés de la présente invention sont également, du fait de leurs propriétés d'inhibition des phosphatases cdc25, susceptibles d'être utilisés pour inhiber la prolifération des microorganismes, notamment des levures. L'un des avantages de ces composés consiste en leur faible toxicité sur les cellules saines.

La demanderesse a donc à présent découvert de façon surprenante que les composés répondant à la formule générale **(I)** 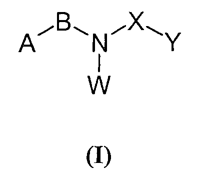 dans laquelle :
A représente un radical **(A1)** 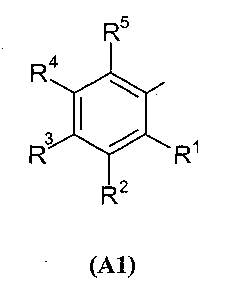
dans lequel deux des groupes R¹, R², R³, R⁴ et R⁵ représentent des atomes d'hydrogène et les trois autres sont choisis indépendamment parmi un atome d'hydrogène, un atome halogène et un radical alkyle, hydroxy, alkoxy, alkylcarbonyloxy, alkylthio ou NR⁶R⁷, étant entendu en outre que :
- ou bien R¹ et l'un de R² et R⁴ sont choisis indépendamment parmi un radical hydroxy, alkylcarbonyloxy et NR⁶R⁷,
- ou bien R² et l'un de R³ et R⁵ sont choisis indépendamment parmi un radical hydroxy, alkylcarbonyloxy et NR⁶R⁷,
- ou bien R⁴ et l'un de R³ et R⁵ sont choisis indépendamment parmi un radical hydroxy, alkylcarbonyloxy et NR⁶R⁷,
- ou encore l'un de R¹, R³ et R⁵ est choisi parmi un radical hydroxy, alkylcarbonyloxy et NR⁶R⁷, et le reste B-N(W)-X-Y est attaché au radical A par un atome d'azote,
R⁶ et R⁷ représentant, indépendamment à chaque fois qu'ils interviennent, un atome d'hydrogène ou un radical alkyle ou R⁶ et R⁷ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR⁸R⁹-, -O-, -S- et -NR¹⁰-, R⁸ et R⁹ représentant indépendamment à chaque fois qu'ils interviennent un atome d'hydrogène ou un radical alkyle, alkoxy, benzyloxycarbonylamino ou dialkylamino, et R¹⁰ représentant indépendamment à chaque fois qu'il intervient un atome d'hydrogène ou un radical alkyle,
ou encore A représente un radical **(A2)** 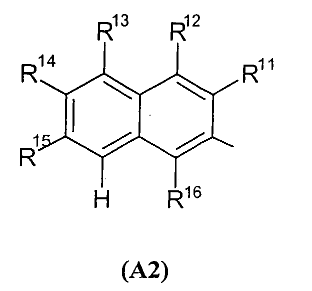 dans lequel :
- ou bien R¹¹ et l'un de R¹³, R¹⁴ et R¹⁵ représentent des radicaux hydroxy tandis que les autres radicaux parmi R¹³, R¹⁴ et R¹⁵ ainsi que R¹⁶ représentent des atomes d'hydrogène,
- ou bien R¹² et R¹⁶ représentent des radicaux hydroxy tandis que R¹¹, R¹³, R¹⁴ et R¹⁵ représentent des atomes d'hydrogène ;
B représente un radical -CO-, -NH-CO-(CH₂)ₙ- ou -(CH₂)ₚ-, n étant un entier de 0 à 3 et p étant un entier de 0 à 1 ;
W représente un atome d'hydrogène ou un radical alkyle ;
X représente un radical -(CH₂)_{q}-, -(CH₂)_{q}-NH- ou -CO-(CH₂)ᵣ-, q étant un entier de 1 à 6 et r un entier de 0 à 6 ;
ou encore l'ensemble B-N(W)-X-Y est tel qu'il représente le radical 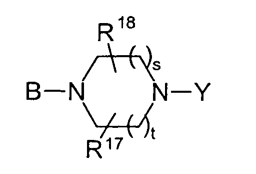 dans lequel B est tel que défini ci-dessus, t est un entier de 0 à 2, s est un entier de 0 à 1 et R¹⁷ et R¹⁸ représentent des radicaux choisis indépendamment parmi un atome d'hydrogène et un radical alkyle ;
et :
- lorsque X représente un radical -(CH₂)_{q}- ou -CO-(CH₂)ᵣ-, alors Y représente un radical 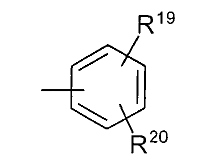
dans lequel R¹⁹ représente un atome d'hydrogène, un atome halogène, un radical nitro, alkyle, alkylthio, NR²¹R²², -SO₂-NR²³R²⁴, -NH-SO₂-R²⁵ ou -O-P(O)(OR²⁶)(OR²⁷),
R²¹ et R²² représentant indépendamment un atome d'hydrogène ou un radical alkyle, R²³ et R²⁴ représentant indépendamment un atome d'hydrogène ou un radical alkyle, ou bien R²³ et R²⁴ représentant ensemble avec l'atome d'azote qui les porte un hétérocycle de 5 à 7 chaînons dont les chaînons complémentaires sont choisis indépendamment parmi -CHR²⁸-, -NR²⁹-, -O- et -S-, R²⁸ et R²⁹ représentant, indépendamment à chaque fois qu'ils interviennent, un atome d'hydrogène ou un radical alkyle,
R²⁵ représentant un radical alkyle, haloalkyle ou l'un des radicaux aryle, hétéroaryle, aralkyle ou hétéroaralkyle dont le noyau aryle ou hétéroaryle est éventuellement substitué par un ou des radicaux choisis indépendamment parmi un atome halogène et des radicaux alkyle, haloalkyle, hydroxy, alkoxy ou nitro, sauf pour les éventuels atomes d'azote du noyau hétéroaryle pour lesquels les substituants éventuels sont choisis parmi des radicaux alkyle,
R²⁶ et R²⁷ étant choisis indépendamment parmi des radicaux alkyle,
et R²⁰ représente un atome d'hydrogène, un atome halogène ou un radical alkyle, alkoxy ou alkylthio,
ou encore Y représente le radical **(T)** représenté ci-dessous 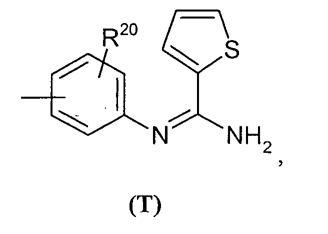 dans lequel R²⁰ représente un atome d'hydrogène ou un radical alkyle, alkoxy ou alkylthio,
- lorsque X représente un radical -(CH₂)_{q}-NH- ou lorsque l'ensemble B-N(W)-X-Y est tel qu'il représente le radical 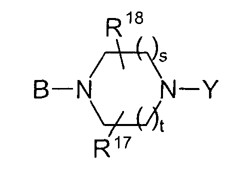 alors Y représente exclusivement un radical -SO₂-R³⁰ dans lequel R³⁰ représente un radical alkyle, haloalkyle ou l'un des radicaux aryle, hétéroaryle, aralkyle ou hétéroaralkyle dont le noyau aryle ou hétéroaryle est éventuellement substitué par un ou des radicaux choisis indépendamment parmi un atome halogène et des radicaux alkyle, haloalkyle, hydroxy, alkoxy ou nitro, sauf pour les éventuels atomes d'azote du noyau hétéroaryle pour lesquels les substituants éventuels sont choisis parmi des radicaux alkyle ;
   étant entendu en outre que lorsque l'ensemble B-N(W)-X-Y est tel qu'il représente le radical 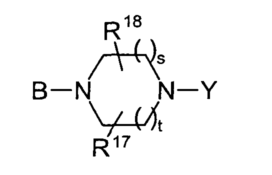 alors B représente exclusivement un radical -CO- ou -(CH₂)- ;
ou les sels pharmaceutiquement acceptables de composés de formule générale **(I)** définie ci-dessus
sont des inhibiteurs de phosphatases cdc25, et en particulier des inhibiteurs de la phosphatase cdc25-C, et peuvent donc être utilisés pour préparer un médicament destiné à inhiber les phosphatases cdc25, et en particulier la phosphatase cdc25-C.

Par alkyle, lorsqu'il n'est pas donné plus de précision, on entend un radical alkyle linéaire ou ramifié comptant de 1 à 12 atomes de carbone, de préférence de 1 à 10 atomes de carbone et plus préférentiellement 1 à 6 atomes de carbone. Par alkényle, lorsqu'il n'est pas donné plus de précision, on entend un radical alkyle linéaire ou ramifié comptant de 1 à 6 atomes de carbone et présentant au moins une insaturation (double liaison). Par alkynyle, lorsqu'il n'est pas donné plus de précision, on entend un radical alkyle linéaire ou ramifié comptant de 1 à 6 atomes de carbone et présentant au moins une double insaturation (triple liaison). Par aryle carbocyclique ou hétérocyclique, on entend un système carbocyclique ou hétérocyclique comprenant au moins un cycle aromatique, un système étant dit hétérocyclique lorsque l'un au moins des cycles qui le composent comporte un hétéroatome (O, N ou S) ; lorsqu'un radical aryle carbocyclique ou hétérocyclique est dit substitué sans qu'il soit donné plus de précision, on entend que ledit radical aryle carbocyclique ou hétérocyclique est substitué de 1 à 3 fois, et de préférence de 1 à 2 fois par des radicaux différents d'un atome d'hydrogène qui, s'ils ne sont pas précisés, sont choisis parmi un atome halogène et les radicaux alkyle ou alkoxy ; par ailleurs, lorsqu'il n'est pas donné plus de précision, on entenr par aryle un aryle carbocyclique exclusivement. Par haloalkyle, on entend un radical alkyle dont au moins l'un des atomes d'hydrogène (et éventuellement tous) est remplacé par un atome halogène.

Par radicaux alkylthio, alkoxy, haloalkyle, haloalkoxy, aminoalkyle, alkényle, alkynyle, aralkyle, on entend respectivement les radicaux alkylthio, alkoxy, haloalkyle, haloalkoxy, aminoalkyle, alkényle, alkynyle, aralkyle dont le radical alkyle a la signification indiquée précédemment.

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par aryle carbocyclique, on entend en particulier les radicaux phényle et naphtyle. Par aryle hétérocyclique ou hétéroaryle, on entend en particulier les radicaux thiényle, imidazolyle, thiazolyle, oxazolyle et pyridyle. Enfin, par halogène, on entend les atomes de fluor, de chlore, de brome ou d'iode.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", *Int. J. Pharm.* (1986), **33**, 201-217.

Dans certains cas, les composés selon la présente invention peuvent comporter des atomes de carbone asymétriques. Par conséquent, les composés selon la présente invention ont deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

De préférence, les composés de formule générale **(I)** selon l'invention incluront au moins l'une des caractéristiques suivantes :
- A représentant un radical **(A1)** 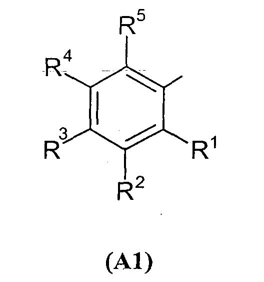
dans lequel deux des groupes R¹, R², R³, R⁴ et R⁵ représentent des atomes d'hydrogène et les trois autres sont choisis indépendamment parmi un atome d'hydrogène, un atome halogène et un radical alkyle, alkylcarbonyloxy, hydroxy, alkoxy ou NR⁶R⁷, étant entendu en outre que :
- ou bien R¹ et l'un de R² et R⁴ sont choisis indépendamment parmi un radical hydroxy, alkylcarbonyloxy et NR⁶R⁷,
- ou bien R² et l'un de R³ et R⁵ sont choisis indépendamment parmi un radical hydroxy, alkylcarbonyloxy et NR⁶R⁷,
- ou bien R⁴ et l'un de R³ et R⁵ sont choisis indépendamment parmi un radical hydroxy, alkylcarbonyloxy et NR⁶R⁷,
- ou encore l'un de R¹, R³ et R⁵ est choisi indépendamment parmi un radical hydroxy, alkylcarbonyloxy et NR⁶R⁷, et le reste B-N(W)-X-Y est attaché au radical A par un atome d'azote,
R⁶ et R⁷ représentant, indépendamment à chaque fois qu'ils interviennent, un atome d'hydrogène ou un radical alkyle ou R⁶ et R⁷ formant ensemble avec l'atome d'azote un hétérocycle de 5 à 7 chaînons comportant de 1 à 2 hétéroatomes, les chaînons nécessaires pour compléter l'hétérocycle étant choisis indépendamment parmi les radicaux -CR⁸R⁹-, -O-, -S- et -NR¹⁰-, R⁸ et R⁹ représentant indépendamment à chaque fois qu'ils interviennent un atome d'hydrogène ou un radical alkyle ou alkoxy, et R¹⁰ représentant indépendamment à chaque fois qu'il intervient un atome d'hydrogène ou un radical alkyle,
ou encore A représentant un radical **(A2)** 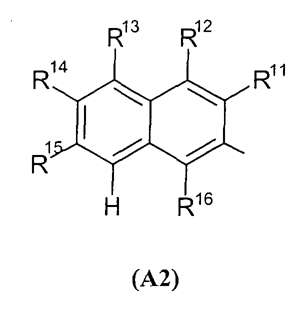 dans lequel :
- ou bien R¹¹ et l'un de R¹³, R¹⁴ et R¹⁵ représentent des radicaux hydroxy tandis que les autres radicaux parmi R¹³, R¹⁴ et R¹⁵ ainsi que R¹⁶ représentent des atomes d'hydrogène,
- ou bien R¹² et R¹⁶ représentent des radicaux hydroxy tandis que R¹¹, R¹³, R¹⁴ et R¹⁵ représentent des atomes d'hydrogène ;

- B représentant un radical -CO-, -NH-CO-(CH₂)ₙ- ou -(CH₂)ₚ-, n étant un entier de 0 à 2 et p étant un entier de 0 à 1 ;
- W représentant un atome d'hydrogène ou un radical alkyle ;
- X représentant un radical -(CH₂)_{q}-, -(CH₂)_{q}-NH- ou -CO-(CH₂)ᵣ, q étant un entier de 1 à 4 et r un entier de 0 à 5 ;
ou encore l'ensemble B-N(W)-X-Y étant tel qu'il représente le radical 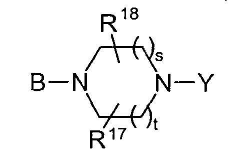 dans lesquels B est tel que défini dans la formule générale **(I),** t est un entier de 0 à 2, s est un entier de 0 à 1, R¹⁷ et R¹⁸ représentent des radicaux choisis indépendamment parmi un atome d'hydrogène et un radical alkyle ;
- lorsque X représente un radical -(CH₂)_{q}- ou -CO-(CH₂)ᵣ-, Y représentant un radical 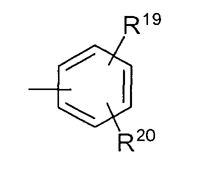 dans lequel R¹⁹ représente un atome d'hydrogène, un atome halogène, un radical nitro, alkyle, alkylthio, NR²¹R²², -SO₂-NR²³R²⁴, -NH-SO₂-R²⁵ ou -O-P(O)(OR²⁶)(OR²⁷),
   R²¹ et R²² représentant indépendamment un atome d'hydrogène ou un radical alkyle,
   R²³ et R²⁴ représentant indépendamment un atome d'hydrogène ou un radical alkyle, ou bien R²³ et R²⁴ représentant ensemble avec l'atome d'azote qui les porte un hétérocycle de 5 à 6 chaînons dont les chaînons complémentaires sont choisis indépendamment parmi -CHR²⁸-, -NR²⁹-, -O- et -S-, R²⁸ et R²⁹ représentant, indépendamment à chaque fois qu'ils interviennent, un atome d'hydrogène ou un radical alkyle,
   R²⁵ représentant un radical alkyle ou aryle éventuellement substitué par un ou des radicaux choisis parmi un atome halogène et des radicaux alkyle, haloalkyle, alkoxy ou nitro, sauf pour les éventuels atomes d'azote du noyau hétéroaryle pour lesquels les substituants éventuels sont choisis parmi des radicaux alkyle,
   R²⁶ et R²⁷ étant choisis indépendamment parmi des radicaux alkyle,
   et R²⁰ représente un atome d'hydrogène ou un radical alkyle ou alkoxy,
   ou encore Y représentant le radical de formule **(T)** 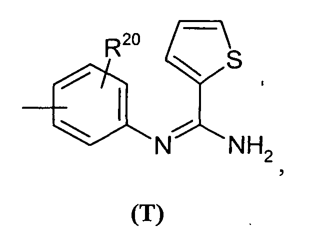 dans lequel R²⁰ représente un atome d'hydrogène ou un radical alkyle ou alkoxy ;
   - lorsque X représente un radical -(CH₂)_{q}-NH- ou lorsque l'ensemble B-N(W)-X-Y est tel qu'il représente le radical 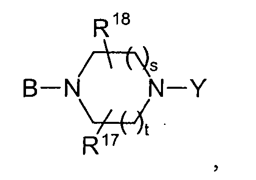 Y représentant un radical -SO₂-R³⁰ dans lequel R³⁰ représente un radical alkyle, haloalkyle ou l'un des radicaux aryle, hétéroaryle, aralkyle ou hétéroaralkyle dont le noyau aryle ou hétéroaryle est éventuellement substitué par un ou des radicaux choisis indépendamment parmi un atome halogène et des radicaux alkyle, haloalkyle, alkoxy ou nitro, sauf pour les éventuels atomes d'azote du noyau hétéroaryle pour lesquels les substituants éventuels sont choisis parmi des radicaux alkyle.

Plus préférentiellement, les composés de formule générale **(I)** selon l'invention incluront au moins l'une des caractéristiques suivantes :
- A représentant un radical **(A1)** 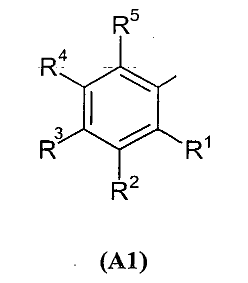
dans lequel deux des groupes R¹, R², R³, R⁴ et R⁵ représentent des atomes d'hydrogène et les trois autres sont choisis indépendamment parmi un atome d'hydrogène, un atome halogène et un radical alkyle, alkylcarbonyloxy, hydroxy, alkoxy ou NR⁶R⁷, étant entendu en outre que :
- ou bien R¹ et l'un de R² et R⁴ sont choisis indépendamment parmi un radical hydroxy, alkylcarbonyloxy et NR⁶R⁷,
- ou bien R² et l'un de R³ et R⁵ sont choisis indépendamment parmi un radical hydroxy, alkylcarbonyloxy et NR⁶R⁷,
- ou bien R⁴ et l'un de R³ et R⁵ sont choisis indépendamment parmi un radical hydroxy, alkylcarbonyloxy et NR⁶R⁷,
- ou encore l'un de R¹, R³ et R⁵ est choisi indépendamment parmi un radical hydroxy, alkylcarbonyloxy et NR⁶R⁷, et le reste B-N(W)-X-Y est attaché au radical A par un atome d'azote,
R⁶ et R⁷ représentant, indépendamment à chaque fois qu'ils interviennent, un atome d'hydrogène ou un radical alkyle comptant de 1 à 3 atomes de carbone ou R⁶ et R⁷ formant ensemble avec l'atome d'azote un hétérocycle de 5 à 6 chaînons comportant de 1 à 2 hétéroatomes, les chaînons nécessaires pour compléter l'hétérocycle étant choisis indépendamment parmi les radicaux -CR⁸R⁹-, -O- et -NR¹⁰-, R⁸ et R⁹ représentant indépendamment à chaque fois qu'ils interviennent un atome d'hydrogène ou un radical alkyle ou alkoxy, et R¹⁰ représentant indépendamment à chaque fois qu'il intervient un atome d'hydrogène ou un radical alkyle,
ou encore A représentant un radical **(A2)** 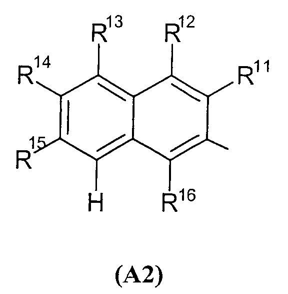 dans lequel :
- ou bien R¹¹ et R¹⁵ représentent des radicaux hydroxy tandis que R¹², R¹³, R¹⁴ et R¹⁶ représentent des atomes d'hydrogène,
- ou bien R¹² et R¹⁶ représentent des radicaux hydroxy tandis que R¹¹, R¹³, R¹⁴ et R¹⁵ représentent des atomes d'hydrogène ;

- W représentant un atome d'hydrogène ou un radical méthyle ou éthyle ;
- X représentant un radical -(CH₂)_{q}-, -(CH₂)_{q}-NH- ou -CO-(CH₂)ᵣ, q étant un entier de 1 à 3 et r un entier de 0 à 4 ;
ou encore l'ensemble B-N(W)-X-Y étant tel qu'il représente le radical 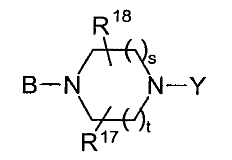 dans lesquels B est tel que défini dans la formule générale **(I)**, t est un entier de 0 à 2, s est un entier de 0 à 1, R¹⁷ et R¹⁸ représentent des radicaux choisis indépendamment parmi un atome d'hydrogène et un radical alkyle comptant de 1 à 3 atomes de carbone ;
- lorsque X représente un radical -(CH₂)_{q}- ou -CO-(CH₂)ᵣ-, Y représentant un radical 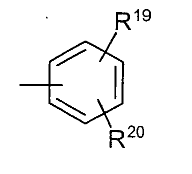
dans lequel R¹⁹ représente un radical nitro, NR²¹R²², -SO₂-NR²³R²⁴, -NH-SO₂-R²⁵ ou -O-P(O)(OR²⁶)(OR²⁷),
R²¹ et R²² représentant indépendamment un atome d'hydrogène ou un radical alkyle,
R²³ et R²⁴ représentant indépendamment un atome d'hydrogène ou un radical alkyle, ou bien R²³ et R²⁴ représentant ensemble avec l'atome d'azote qui les porte un hétérocycle de 5 à 6 chaînons dont les chaînons complémentaires sont choisis indépendamment parmi -CHR²⁸-, -NR²⁹-, -O- et -S-, R²⁸ et R²⁹ représentant, indépendamment à chaque fois qu'ils interviennent, un atome d'hydrogène ou un radical alkyle,
R²⁵ représentant un radical alkyle ou aryle éventuellement substitué par un ou des radicaux choisis parmi un atome halogène et des radicaux alkyle, haloalkyle, alkoxy ou nitro, sauf pour les éventuels atomes d'azote du noyau hétéroaryle pour lesquels les substituants éventuels sont choisis parmi des radicaux alkyle,
R²⁶ et R²⁷ étant choisis indépendamment parmi des radicaux alkyle,
et R²⁰ représente un atome d'hydrogène ou un radical alkyle,
ou encore Y représentant le radical de formule **(T)** 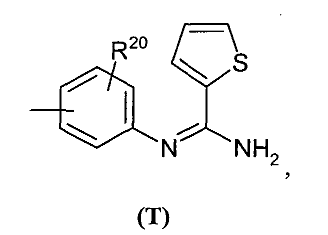 dans lequel R²⁰ représente un atome d'hydrogène ou un radical alkyle ;
- lorsque X représente un radical -(CH₂)_{q}-NH- ou lorsque l'ensemble B-N(W)-X-Y est tel qu'il représente le radical 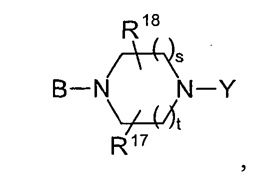 Y représentant un radical -SO₂-R³⁰ dans lequel R³⁰ représente un radical alkyle, haloalkyle ou l'un des radicaux aryle ou aralkyle dont le noyau aryle est éventuellement substitué par un ou des radicaux choisis indépendamment parmi un atome halogène et des radicaux alkyle, haloalkyle, alkoxy ou nitro, sauf pour les éventuels atomes d'azote du noyau hétéroaryle pour lesquels les substituants éventuels sont choisis parmi des radicaux alkyle.

Encore plus préférentiellement, les composés de formule générale **(I)** selon l'invention incluront au moins l'une des caractéristiques suivantes :
- A représentant un radical **(A1)** 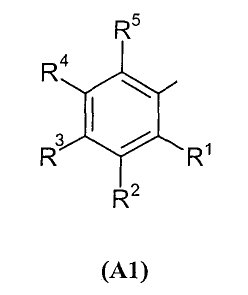 dans lequel deux des groupes R¹, R², R³, R⁴ et R⁵ représentent des atomes d'hydrogène et les trois autres sont choisis indépendamment parmi un atome d'hydrogène, un atome halogène et un radical alkyle, acétoxy, hydroxy, méthoxy ou NR⁶R⁷, étant entendu en outre que :
   - ou bien R¹ et l'un de R² et R⁴ sont choisis indépendamment parmi un radical hydroxy, acétoxy et NR⁶R⁷,
   - ou bien R² et l'un de R³ et R⁵ sont choisis indépendamment parmi un radical hydroxy, acétoxy et NR⁶R⁷,
   - ou bien R⁴ et l'un de R³ et R⁵ sont choisis indépendamment parmi un radical hydroxy, acétoxy et NR⁶R⁷,
   - ou encore l'un de R¹, R³ et R⁵ est choisi indépendamment parmi un radical hydroxy, acétoxy et NR⁶R⁷, et le reste B-N(W)-X-Y est attaché au radical A par un atome d'azote,
R⁶ et R⁷ représentant, indépendamment à chaque fois qu'ils interviennent, un atome d'hydrogène ou un radical alkyle comptant de 1 à 3 atomes de carbone (ce radical alkyle étant de préférence le radical méthyle) ou R⁶ et R⁷ formant ensemble avec l'atome d'azote un hétérocycle comptant 6 chaînons et comportant de 1 à 2 hétéroatomes, les chaînons nécessaires pour compléter l'hétérocycle étant choisis indépendamment parmi les radicaux -CR⁸R⁹-, -O- et -NR¹⁰-, R⁸ et R⁹ représentant indépendamment à chaque fois qu'ils interviennent un atome d'hydrogène ou un radical alkyle (ce radical alkyle étant de préférence le radical méthyle), et R¹⁰ représentant indépendamment à chaque fois qu'il intervient un atome d'hydrogène ou un radical alkyle (ce radical alkyle étant de préférence le radical méthyle),
ou encore A représentant un radical **(A2)** 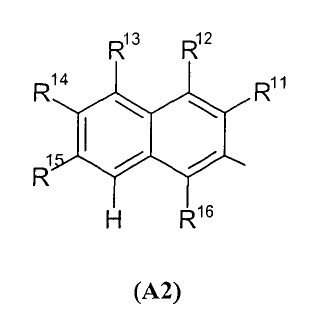 dans lequel R¹¹ et R¹⁵ représentent des radicaux hydroxy tandis que R¹², R¹³, R¹⁴ et
R¹⁶ représentent des atomes d'hydrogène ;
- W représentant un atome d'hydrogène ou un radical méthyle ;
- X représentant un radical -(CH₂)_{q}-, -(CH₂)_{q}-NH- ou -CO-(CH₂)ᵣ, q étant un entier de 1 à 3 et r un entier de 0 à 4 ;
   ou encore l'ensemble B-N(W)-X-Y étant tel qu'il représente le radical 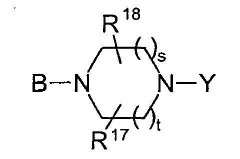 dans lesquels B est tel que défini dans la formule générale **(I),** t est un entier de 0 à 2, s est un entier de 0 à 1, R¹⁷ et R¹⁸ représentent des radicaux choisis indépendamment parmi un atome d'hydrogène et un radical méthyle ;
- lorsque X représente un radical -(CH₂)_{q}- ou -CO-(CH₂)ᵣ-, Y représentant un radical 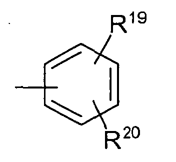 dans lequel R¹⁹ représente un radical nitro, NR²¹R²², -SO₂-NR²³R²⁴, -NH-SO₂-R²⁵ ou -O-P(O)(OR²⁶)(OR²⁷),
   R²¹ et R²² représentant indépendamment un atome d'hydrogène ou un radical alkyle,
   R²³ et R²⁴ représentant indépendamment un atome d'hydrogène ou un radical alkyle, ou bien R²³ et R²⁴ représentant ensemble avec l'atome d'azote qui les porte un hétérocycle de 5 à 6 chaînons dont les chaînons complémentaires sont choisis indépendamment parmi -CHR²⁸-, -NR²⁹-, -O- et -S-, R²⁸ et R²⁹ représentant, indépendamment à chaque fois qu'ils interviennent, un atome d'hydrogène ou un radical alkyle,
   R²⁵ représentant un radical alkyle ou aryle éventuellement substitué par un ou des radicaux choisis parmi un atome halogène et des radicaux alkyle, haloalkyle, alkoxy ou nitro, sauf pour les éventuels atomes d'azote du noyau hétéroaryle pour lesquels les substituants éventuels sont choisis parmi des radicaux alkyle,
   R²⁶ et R²⁷ étant choisis indépendamment parmi des radicaux alkyle,
   et R²⁰ représente un atome d'hydrogène ou un radical méthyle (et de préférence un atome d'hydrogène),
   ou encore Y représentant le radical de formule **(T)** 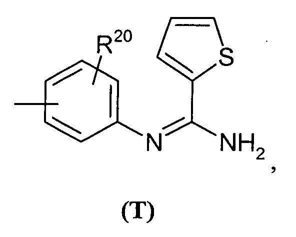 dans lequel R²⁰ représente un atome d'hydrogène ou un radical méthyle (et de préférence un atome d'hydrogène) ;
- lorsque X représente un radical -(CH₂)_{q}-NH- ou lorsque l'ensemble B-N(W)-X-Y est tel qu'il représente le radical 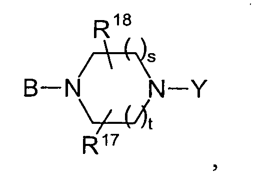 Y représentant un radical -SO₂-R³⁰ dans lequel R³⁰ représente un radical alkyle ou l'un des radicaux aryle ou aralkyle dont le noyau aryle est éventuellement substitué par un ou des radicaux choisis indépendamment parmi un atome halogène et des radicaux alkyle, haloalkyle, alkoxy ou nitro, sauf pour les éventuels atomes d'azote du noyau hétéroaryle pour lesquels les substituants éventuels sont des radicaux méthyle.

Seront particulièrement préférés pour une utilisation selon l'invention les composés suivants décrits (le cas échéant sous forme de sels) dans les exemples :
- 4-(diméthylamino)-2-méthoxy-6-({méthyl[2-(4-nitrophényl)éthyl]amino}méthyl)-phénol ;
- 4-(diméthylamino)-2-({méthyl[2-(4-nitrophényl)éthyl]amino}méthyl)phénol ;
- 2,7-dihydroxy-N-{2-[4-[(2-thiényl(imino)méthyl)amino]phényl]éthyl}-2-napthalènecarboxamide;
- 3-[(3- {[amino(2-thiényl)méthylidène]amino}-benzyl)amino]-N-[4-(diméthylamino) phényl]propanamide ;
- 4-(4-aminophényl)-*N*-[4-(4-méthyl-1-pipérazinyl)phényl]butanamide ;
- 4-(diméthylamino)-2-méthoxy-6-({[2-(4-nitrophényl)éthyl]amino}méthyl)phénol ;
- 4-(diméthylamino)-2-({[2-(4-nitrophényl)éthyl]amino}méthyl)phénol ;
- 2-(diméthylamino)-6-méthoxy-4-({méthyl[2-(4-nitrophényl)éthyl]amino}méthyl) phénol ;
- 2-({méthyl[2-(4-nitrophényl)éthyl]amino}méthyl)-1,4-benzènediol ;
- acétate de 4-(diméthylamino)-2-méthoxy-6-({méthyl[2-(4-nitrophényl)éthyl]amino} méthyl)phényle ;
- 3,7-dihydroxy-*N*-[2-(4-nitrophényl)éthyl]-2-naphthamide ;
- *N*-[4-(diméthylamino)benzyl]-3,7-dihydroxy-2-naphthamide ;
- 4-{2-[(3,7-dihydroxy-2-naphthoyl)amino]éthyl}phénylphosphate de diéthyle ;
- *N*-{2-[4-(aminosulfonyl)phényl]éthyl}-3,7-dihydroxy-2-naphthamide ;
- 3,7-dihydroxy-*N*-[2-(4-aminophényl)éthyl]-2-naphthamide ;
- 3,7-dihydroxy-*N*-(2-{4-[(méthylsulfonyl)amino]phényl}éthyl)-2-naphthamide ;
- *N*-(2-{4-[(butylsulfonyl)amino]phényl}éthyl)-3,7-dihydroxy-2-naphthamide ;
- 3,7-dihydroxy-*N*-[2-(4-{[(4-méthylphényl)sulfonyl]amino}phényl)éthyl]-2-naphthamide ;
- 3,7-dihydroxy-*N*-(2-{4-[(1-naphthylsulfonyl)amino]phényl}éthyl)-2-naphthamide ;
- 3,7-dihydroxy-*N*-{2-[4-({[2-(trifluorométhyl)phényl]sulfonyl}amino)phényl]éthyl}-2-naphthamide ;
- *N*-(2-{4-[(benzylsulfonyl)amino]phényl}éthyl)-3,7-dihydroxy-2-naphthamide ;
- 3,7-dihydroxy-*N*-{2-[4-({[3-(trifluorométhyl)phényl]sulfonyl}amino)phényl]éthyl}-2-naphthamide ;
- 3,7-dihydroxy-*N*-[2-(4-{[(4-nitrophényl)sulfonyl]amino}phényl)éthyl]-2-naphthamide ;
- 3,7-dihydroxy-*N*-{2-[4-({[4-(trifluorométhyl)phényl]sulfonyl}amino) phényl]éthyl}-2-naphthamide ;
- 3,7-dihydroxy-*N*-(2-{4-[(thién-2-ylsulfonyl)amino]phényl}éthyl)-2-naphthamide ;
- 3,7-dihydroxy-*N*-[2-(4-{[(4-méthoxyphényl)sulfonyl]amino}phényl)éthyl]-2-naphthamide ;
- 3,7-dihydroxy-*N*-[2-(4-{[(1-méthyl-1H-imidazol-4-yl)sulfonyl]amino}phényl)éthyl]-2-naphthamide ;
- *N*-[2-(4-{[(4-fluorophényl)sulfonyl]amino}phényl)éthyl]-3,7-dihydroxy-2-naphthamide ;
- 3,7-dihydroxy-*N*-{3-[(4-méthyl-1-pipéridinyl)sulfonyl]benzyl}-2-naphthamide ;
- 5-(4-{[(1*E*)-amino(2-thiényl)méthylidène]amino}phényl)-N-[2-(diméthylamino) phényl]pentanamide ;
- 3-({4-[(4-méthylphényl)sulfonyl]pipérazin-1-yl}carbonyl)naphthalène-2,6-diol ;
- 3-{[4-(méthylsulfbnyl)pipérazin-l-yl]carbonyl}naphthalène-2,6-diol ;
- 3-{[4-(butylsulfonyl)pipérazin-1-yl]carbonyl}naphthalène-2,6-diol ;
ou les sels pharmaceutiquement acceptables de tels composés.

Les composés suivants seront encore plus particulièrement préférés encore pour une utilisation selon l'invention :
- 4-(diméthylamino)-2-méthoxy-6-({méthyl[2-(4-nitrophényl)éthyl]amino}méthyl)-phénol;
- 4-(diméthylamino)-2-({méthyl[2-(4-nitrophényl)éthyl]amino}méthyl)phénol ;
- 2-(diméthylamino)-6-méthoxy-4-({méthyl[2-(4-nitrophényl)éthyl]amino}méthyl) phénol ;
- 2-({méthyl[2-(4-nitrophényl)éthyl]amino}méthyl)-1,4-benzènediol ;
- acétate de 4-(diméthylamino)-2-méthoxy-6-({méthyl[2-(4-nitrophényl)éthyl]amino} méthyl)phényle ;
- 4-{2-[(3,7-dihydroxy-2-naphthoyl)amino]éthyl}phénylphosphate de diéthyle ;
- *N*-{2-[4-(aminosulfonyl)phényl]éthyl}-3,7-dihydroxy-2-naphthamide ;
- 3,7-dihydroxy-*N*-(2-{4-[(méthylsulfonyl)amino]phényl}éthyl)-2-naphthamide ;
- *N*-(2-{4-[(butylsulfonyl)amino]phényl}éthyl)-3,7-dihydroxy-2-naphthamide ;
- 3,7-dihydroxy-*N*-[2-(4-{[(4-méthylphényl)sulfonyl]amino}phényl)éthyl]-2-naphthamide ;
- 3,7-dihydroxy-*N*-{2-[4-({[3-(trifluorométhyl)phényl]sulfonyl}amino)phényl]éthyl}-2-naphthamide ;
- *N*-[2-(4-{[(4-fluorophényl)sulfonyl]amino}phényl)éthyl]-3,7-dihydroxy-2-naphthamide ;
- 3,7-dihydroxy-*N*-{3-[(4-méthyl-1-pipéridinyl)sulfonyl]benzyl}-2-naphthamide ;
- 3-({4-[(4-méthylphényl)sulfonyl]pipérazin-1-yl}carbonyl)naphthalène-2,6-diol ;
ou les sels pharmaceutiquement acceptables de tels composés.

Par ailleurs, le 5-(4-{[(1*E*)-amino(2-thiényl)méthylidène]amino}phényl)-N-[2-(diméthylamino)phényl]pentanamide et ses sels pharmaceutiquement acceptables seront aussi préférés pour une utilisation selon l'invention.

Seront tout particulièrement préférés pour une utilisation selon l'invention les composés suivants :
- 4-(diméthylamino)-2-méthoxy-6-({méthyl[2-(4-nitrophényl)éthyl]amino}méthyl)-phénol ;
- 4-(diméthylamino)-2-({méthyl[2-(4-nitrophényl)éthyl]amino}méthyl)phénol ;
- 2-({méthyl[2-(4-nitrophényl)éthyl]amino}méthyl)-1,4-benzènediol ;
- acétate de 4-(diméthylamino)-2-méthoxy-6-({méthyl[2-(4-nitrophényl)éthyl]amino} méthyl)phényle ;
- 3,7-dihydroxy-*N*-{2-[4-({[3-(trifluorométhyl)phényl]sulfonyl}amino)phényl]éthyl}-2-naphthamide ;
- 3,7-dihydroxy-*N*-{3-[(4-méthyl-1-pipéridinyl)sulfonyl]benzyl}-2-naphthamide ;
ou les sels pharmaceutiquement acceptables de tels composés.

De préférence, les composés de formule générale **(I)** seront utilisés pour préparer un médicament destiné à traiter une maladie choisie parmi les maladies suivantes : les maladies prolifératives tumorales, et en particulier le cancer, les maladies prolifératives non tumorales, les maladies parasitaires, les infections virales, l'alopécie spontanée, l'alopécie induite par des produits exogènes et l'alopécie radio-induite.

De préférence toutefois, les composés de formule générale **(I)** utilisés pour préparer un médicament destiné à traiter les maladies prolifératives, les maladies parasitaires et les infections virales seront tels que le radical Y ne représente pas le radical de formule **(T)**. les composés de formule générale **(I)** dans lesquels le radical Y représente le radical de formule **(T)** seront donc de préférence utilisés pour préparer un médicament destiné à traiter l'alopécie spontanée, l'alopécie induite par des produits exogènes et l'alopécie radio-induite.

Tout particulièrement, les composés de formule générale **(I)** pourront être utilisés pour préparer un médicament destiné à traiter le cancer, et notamment la cancer du sein, les lymphomes, les cancers du cou et de la tête, le cancer du poumon, le cancer du colon, le cancer de la prostate et le cancer du pancréas.

Selon une variante particulière de l'invention, les composés de formule générale **(I)** tels que définis ci-dessus peuvent être utilisés pour préparer un médicament destiné à traiter l'alopécie spontanée, l'alopécie induite par des produits exogènes ou l'alopécie radio-induite.

La présente invention offre aussi, à titre de médicaments, les composés de formule générale **(II)** 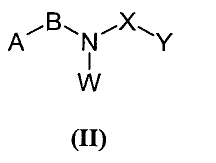 dans laquelle
A représente un radical **(A1)** 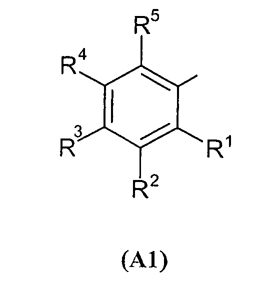 dans lequel deux des groupes R¹, R², R³, R⁴ et R⁵ représentent des atomes d'hydrogène et les trois autres sont choisis indépendamment parmi un atome d'hydrogène, un atome halogène et un radical alkyle, hydroxy, alkoxy, alkylcarbonyloxy, alkylthio ou NR⁶R⁷, étant entendu en outre que :
- ou bien R¹ et l'un de R² et R⁴ sont choisis indépendamment parmi un radical hydroxy, alkylcarbonyloxy et NR⁶R⁷,
- ou bien R² et l'un de R³ et R⁵ sont choisis indépendamment parmi un radical hydroxy, alkylcarbonyloxy et NR⁶R⁷,
- ou bien R⁴ et l'un de R³ et R⁵ sont choisis indépendamment parmi un radical hydroxy, alkylcarbonyloxy et NR⁶R⁷,
- ou encore l'un de R¹, R³ et R⁵ est choisi parmi un radical hydroxy, alkylcarbonyloxy et NR⁶R⁷, et le reste B-N(W)-X-Y est attaché au radical A par un atome d'azote,
R⁶ et R⁷ représentant, indépendamment à chaque fois qu'ils interviennent, un atome d'hydrogène ou un radical alkyle ou R⁶ et R⁷ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR⁸R⁹-, -O-, -S- et -NR¹⁰-, R⁸ et R⁹ représentant indépendamment à chaque fois qu'ils interviennent un atome d'hydrogène ou un radical alkyle, alkoxy, benzyloxycarbonylamino ou dialkylamino, et R¹⁰ représentant indépendamment à chaque fois qu'il intervient un atome d'hydrogène ou un radical alkyle,
ou encore A représente un radical **(A2)** 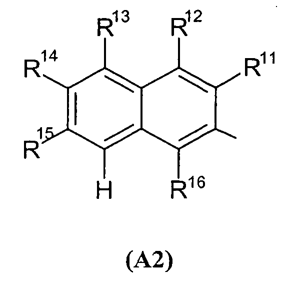 dans lequel :
- ou bien R¹¹ et l'un de R¹³, R¹⁴ et R¹⁵ représentent des radicaux hydroxy tandis que les autres radicaux parmi R¹³, R¹⁴ et R¹⁵ ainsi que R¹⁶ représentent des atomes d'hydrogène,
- ou bien R¹² et R¹⁶ représentent des radicaux hydroxy tandis que R¹¹, R¹³, R¹⁴ et R¹⁵ représentent des atomes d'hydrogène ;
B représente un radical -CO-, -NH-CO-(CH₂)ₙ- ou -(CH₂)ₚ-, n étant un entier de 0 à 3 et p étant un entier de 0 à 1 ;
W représente un atome d'hydrogène ou un radical alkyle ;
X représenté un radical -(CH₂)_{q}-, -(CH₂)_{q}-NH- ou -CO-(CH₂)ᵣ-, q étant un entier de 1 à 6 et r un entier de 0 à 6 ;
ou encore l'ensemble B-N(W)-X-Y est tel qu'il représente le radical 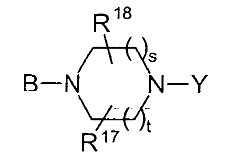 dans lequel B est tel que défini ci-dessus, t est un entier de 0 à 2, s est un entier de 0 à 1 et R¹⁷ et R¹⁸ représentent des radicaux choisis indépendamment parmi un atome d'hydrogène et un radical alkyle ;
et :
- lorsque X représente un radical -(CH₂)_{q}- ou -CO-(CH₂)ᵣ-, alors Y représente un radical 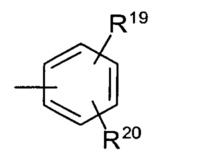
dans lequel R¹⁹ représente un atome d'hydrogène, un atome halogène, un radical nitro, alkyle, alkylthio, NR²¹R²², -SO₂-NR²³R²⁴, -NH-SO₂-R²⁵ ou -O-P(O)(OR²⁶)(OR²⁷),
R²¹ et R²² représentant indépendamment un atome d'hydrogène ou un radical alkyle,
R²³ et R²⁴ représentant indépendamment un atome d'hydrogène ou un radical alkyle, ou bien R²³ et R²⁴ représentant ensemble avec l'atome d'azote qui les porte un hétérocycle de 5 à 7 chaînons dont les chaînons complémentaires sont choisis indépendamment parmi -CHR²⁸-, -NR²⁹-, -O- et -S-, R²⁸ et R²⁹ représentant, indépendamment à chaque fois qu'ils interviennent, un atome d'hydrogène ou un radical alkyle,
R²⁵ représentant un radical alkyle, haloalkyle ou l'un des radicaux aryle, hétéroaryle, aralkyle ou hétéroaralkyle dont le noyau aryle ou hétéroaryle est éventuellement substitué par un ou des radicaux choisis indépendamment parmi un atome halogène et des radicaux alkyle, haloalkyle, hydroxy, alkoxy ou nitro, sauf pour les éventuels atomes d'azote du noyau hétéroaryle pour lesquels les substituants éventuels sont choisis parmi des radicaux alkyle,
R²⁶ et R²⁷ étant choisis indépendamment parmi des radicaux alkyle,
et R²⁰ représente un atome d'hydrogène, un atome halogène ou un radical alkyle, alkoxy ou alkylthio ;
- lorsque X représente un radical -(CH₂)_{q}-NH- ou lorsque l'ensemble B-N(W)-X-Y est tel qu'il représente le radical 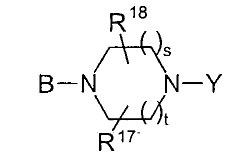 alors Y représente exclusivement un radical -SO₂-R³⁰ dans lequel R³⁰ représente un radical alkyle, haloalkyle ou l'un des radicaux aryle, hétéroaryle, aralkyle ou hétéroaralkyle dont le noyau aryle ou hétéroaryle est éventuellement substitué par un ou des radicaux choisis indépendamment parmi un atome halogène et des radicaux alkyle, haloalkyle, hydroxy, alkoxy ou nitro, sauf pour les éventuels atomes d'azote du noyau hétéroaryle pour lesquels les substituants éventuels sont choisis parmi des radicaux alkyle ;
   étant entendu en outre que lorsque l'ensemble B-N(W)-X-Y est tel qu'il représente le radical 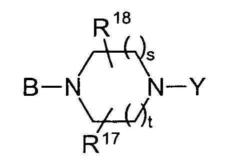 alors B représente exclusivement un radical -CO- ou -(CH₂)- ;
ou les sels pharmaceutiquement acceptables de composés de formule générale **(II).**

En particulier, l'invention concerne, à titre de médicaments, les composés de formule générale **(II)** suivants :
- 4-(diméthylamino)-2-méthoxy-6-({méthyl[2-(4-nitrophényl)éthyl]amino}méthyl) phénol ;
- 4-(diméthylamino)-2-({méthyl[2-(4-nitrophényl)éthyl]amino}méthyl)phénol ;
- 4-(4-aminophényl)-*N*-[4-(4-méthyl-1-pipérazinyl)phényl]butanamide ;
- 4-(diméthylamino)-2-méthoxy-6-({[2-(4-nitrophényl)éthyl]amino}méthyl)phénol ;
- 4-(diméthylamino)-2-({[2-(4-nitrophényl)éthyl]amino}méthyl)phénol ;
- 2-(diméthylamino)-6-méthoxy-4-({méthyl[2-(4-nitrophényl)éthyl]amino}méthyl) phénol ;
- 2-({méthyl[2-(4-nitrophényl)éthyl]amino}méthyl)-1,4-benzènediol ;
- acétate de 4-(diméthylamino)-2-méthoxy-6-({méthyl[2-(4-nitrophényl)éthyl]amino} méthyl)phényle ;
- 3,7-dihydroxy-*N*-[2-(4-nitrophényl)éthyl]-2-naphthamide ;
- *N*-[4-(diméthylamino)benzyl]-3,7-dihydroxy-2-naphthamide ;
- 4-{2-[(3,7-dihydroxy-2-naphthoyl)amino]éthyl}phénylphosphate de diéthyle ;
- *N*-{2-[4-(aminosulfonyl)phényl]éthyl}-3,7-dihydroxy-2-naphthamide ;
- 3,7-dihydroxy-*N*-[2-(4-aminophényl)éthyl]-2-naphthamide ;
- 3,7-dihydroxy-*N*-(2-{4-[(méthylsulfonyl)amino]phényl}éthyl)-2-naphthamide ;
- *N*-(2-{4-[(butylsulfonyl)amino]phényl}éthyl)-3,7-dihydroxy-2-naphthamide ;
- 3,7-dihydroxy-*N*-[2-(4-{[(4-méthylphényl)sulfonyl]amino}phényl)éthyl]-2-naphthamide ;
- 3,7-dihydroxy-*N*-(2-{4-[(1-naphthylsulfonyl)amino]phényl}éthyl)-2-naphthamide ;
- 3,7-dihydroxy-*N*-{2-[4-({[2-(trifluorométhyl)phényl]sulfonyl}amino)phényl]éthyl}-2-naphthamide ;
- *N*-(2-{4-[(benzylsulfonyl)amino]phényl)éthyl)-3,7-dihydroxy-2-naphthamide ;
- 3,7-dihydroxy-*N*-{2-[4-({[3-(trifluorométhyl)phényl]sulfonyl}amino)phényl]éthyl}-2-naphthamide ;
- 3,7-dihydroxy-*N*-[2-(4-{[(4-nitrophényl)sulfonyl]amino}phényl)éthyl]-2-naphthamide ;
- 3,7-dihydroxy-*N*-{2-[4-({[4-(trifluorométhyl)phényl]sulfonyl}amino)phényl]éthyl}-2-naphthamide ;
- 3,7-dihydroxy-*N*-(2-{4-[(thién-2-ylsulfonyl)amino]phényl}éthyl)-2-naphthamide ;
- 3,7-dihydroxy-*N*-[2-(4-{[(4-méthoxyphényl)sulfonyl]amino}phényl)éthyl]-2-naphthamide ;
- 3,7-dihydroxy-*N*-[2-(4-{[(1-méthyl-1H-imidazol-4-yl)sulfonyl]amino}phényl)éthyl]-2-naphthamide ;
- *N*-[2-(4-{[(4-fluorophényl)sulfonyl]amino}phényl)éthyl]-3,7-dihydroxy-2-naphthamide ;
- 3,7-dihydroxy-*N*-{3-[(4-méthyl-1-pipéridinyl)sulfonyl]benzyl}-2-naphthamide ;
- 3-({4-[(4-méthylphényl)sulfonyl]pipérazin-1-yl}carbonyl)naphthalène-2,6-diol ;
- 3- {[4-(méthylsulfonyl)pipérazin-1-yl]carbonyl}naphthalène-2,6-diol ;
- 3-{[4-(butylsulfonyl)pipérazin-1-yl]carbonyl}naphthalène-2,6-diol ;
et leurs sels pharmaceutiquement acceptables.

L'invention concerne par ailleurs aussi, à titre de médicament, le 5-(4-{[(1*E*)-amino (2-thiényl)méthylidène]amino}phényl)-N-[2-(dimethylamino) phényl]pentanamide ou un de ses sels pharmaceutiquement acceptables.

L'invention concerne de plus les compositions pharmaceutiques comprenant, à titre de principe actif, au moins un des composés de formule générale **(II)** définie ci-dessus ou un sel pharmaceutiquement acceptable d'un tel composé, et de préférence un composé choisi parmi les composés suivants :
- 4-(diméthylamino)-2-méthoxy-6-({méthyl[2-(4-nitrophényl)éthyl]amino}méthyl) phénol ;
- 4-(diméthylamino)-2-({méthyl[2-(4-nitrophényl)éthyl]amino}méthyl)phénol ;
- 4-(4-aminophényl)-*N*-[4-(4-méthyl-1-pipérazinyl)phényl]butanamide ;
- 4-(diméthylamino)-2-méthoxy-6-({[2-(4-nitrophényl)éthyl]amino}méthyl)phénol ;
- 4-(diméthylamino)-2-({[2-(4-nitrophényl)éthyl]amino}méthyl)phénol ;
- 2-(diméthylamino)-6-méthoxy-4-({méthyl[2-(4-nitrophényl)éthyl]amino}méthyl) phénol ;
- 2-({méthyl[2-(4-nitrophényl)éthyl]amino}méthyl)-1,4-benzènediol ;
- acétate de 4-(diméthylamino)-2-méthoxy-6-({méthyl[2-(4-nitrophényl)éthyl]amino} méthyl)phényle ;
- 3,7-dihydroxy-*N*-[2-(4-nitrophényl)éthyl]-2-naphthamide ;
- *N*-[4-(diméthylamino)benzyl]-3,7-dihydroxy-2-naphthamide ;
- 4-{2-[(3,7-dihydroxy-2-naphthoyl)amino]éthyl}phénylphosphate de diéthyle ;
- *N*-{2-[4-(aminosulfonyl)phényl]éthyl}-3,7-dihydroxy-2-naphthamide ;
- 3,7-dihydroxy-*N*-[2-(4-aminophényl)éthyl]-2-naphthamide ;
- 3,7-dihydroxy-*N*-(2-{4-[(méthylsulfonyl)amino]phényl}éthyl)-2-naphthamide ;
- *N*-(2-{4-[(butylsulfonyl)amino]phényl}éthyl)-3,7-dihydroxy-2-naphthamide ;
- 3,7-dihydroxy-*N*-[2-(4-{[(4-méthylphényl)sulfonyl]amino}phényl)éthyl]-2-naphthamide ;
- 3,7-dihydroxy-*N*-(2-{4-[(1-naphthylsulfonyl)amino]phényl}éthyl)-2-naphthamide ;
- 3,7-dihydroxy-*N*-{2-[4-({[2-(trifluorométhyl)phényl]sulfonyl}amino)phényl]éthyl}-2-naphthamide ;
- *N*-(2-{4-[(benzylsulfonyl)amino]phényl}éthyl)-3,7-dihydroxy-2-naphthamide ;
- 3,7-dihydroxy-*N*-{2-[4-({[3-(trifluorométhyl)phényl]sulibnyl} amino)phényl]éthyl}-2-naphthamide ;
- 3,7-dihydroxy-*N*-[2-(4-{[(4-nitrophényl)sulfonyl]amino}phényl)éthyl]-2-naphthamide ;
- 3,7-dihydroxy-*N*-{2-[4-({[4-(trifluorométhyl)phényl]sulfonyl}amino)phényl]éthyl}-2-naphthamide ;
- 3,7-dihydroxy-*N*-(2-{4-[(thién-2-ylsulfonyl)amino]phényl}éthyl)-2-naphthamide ;
- 3,7-dihydroxy-*N*-[2-(4-{[(4-méthoxyphényl)sulfonyl]amino}phényl)éthyl]-2-naphthamide ;
- 3,7-dihydroxy-*N*-[2-(4-{[(1-méthyl-1H-imidazol-4-yl)sulfonyl]amino}phényl)éthyl]-2-naphthamide ;
- *N*-[2-(4-{[(4-fluorophényl)sulfonyl]amino}phényl)éthyl]-3,7-dihydroxy-2-naphthamide ;
- 3,7-dihydroxy-*N*-{3-[(4-méthyl-1-pipéridinyl)sulfonyl]benzyl}-2-naphthamide ;
- 3-({4-[(4-méthylphényl)sulfonyl]pipérazin-1-yl}carbonyl)naphthalène-2,6-diol ;
- 3-{[4-(méthylsulfonyl)pipérazin-1-yl]carbonyl}naphthalène-2,6-diol ;
- 3-{[4-(butylsulfonyl)pipérazin-1-yl]carbonyl}naphthalène-2,6-diol ;
et leurs sels pharmaceutiquement acceptables.

Selon une variante de l'invention, une composition pharmaceutique selon l'invention comprendra du 5-(4-{[(1*E*)-amino(2-thiényl)méthylidène]amino}phényl)-N-[2-(diméthylamino) phényl]pentanamide ou un de ses sels pharmaceutiquement acceptables.

Plus préférentiellement, une composition pharmaceutique selon l'invention comprendra, à titre de principe actif, un composé choisi parmi les composés suivants :
- 4-(diméthylamino)-2-méthoxy-6-({méthyl[2-(4-nitrophényl)éthyl]amino}méthyl)-phénol ;
- 4-(diméthylamino)-2-({méthyl[2-(4-nitrophényl)éthyl]amino}méthyl)phénol ;
- 2-(diméthylamino)-6-méthoxy-4-({méthyl[2-(4-nitrophényl)éthyl]amino}méthyl) phénol ;
- 2-({méthyl[2-(4-nitrophényl)éthyl]amino}méthyl)-1,4-benzènediol ;
- acétate de 4-(diméthylamino)-2-méthoxy-6-({méthyl[2-(4-nitrophényl)éthyl]amino} méthyl)phényle ;
- 4-{2-[(3,7-dihydroxy-2-naphthoyl)amino]éthyl}phénylphosphate de diéthyle ;
- *N*-{2-[4-(aminosulfonyl)phényl]éthyl}-3,7-dihydroxy-2-naphthamide ;
- 3,7-dihydroxy-*N*-(2-{4-[(méthylsulfonyl)amino]phényl}éthyl)-2-naphthamide ;
- *N*-(2-{4-[(butylsulfonyl)amino]phényl}éthyl)-3,7-dihydroxy-2-naphthamide ;
- 3,7-dihydroxy-*N*-[2-(4-{[(4-méthylphényl)sulfonyl]amino}phényl)éthyl]-2-naphthamide ;
- 3,7-dihydroxy-*N*-{2-[4-({[3-(trifluorométhyl)phényl]sulfonyl}amino)phényl]éthyl}-2-naphthamide ;
- *N*-[2-(4-{[(4-fluorophényl)sulfonyl]amino}phényl)éthyl]-3,7-dihydroxy-2-naphthamide ;
- 3,7-dihydroxy-*N*-{3-[(4-méthyl-1-pipéridinyl)sulfonyl]benzyl}-2-naphthamide ;
- 3-({4-[(4-méthylphényl)sulfonyl]pipérazin-1-yl}carbonyl)naphthalène-2,6-diol ;
ou un sel pharmaceutiquement acceptable d'un de ces derniers.

Encore plus préférentiellement, une composition pharmaceutique selon l'invention comprendra, à titre de principe actif, un composé choisi parmi les composés suivants :
- 4-(diméthylamino)-2-méthoxy-6-({méthyl[2-(4-nitrophényl)éthyl]amino}méthyl)-phénol;
- 4-(diméthylamino)-2-({méthyl[2-(4-nitrophényl)éthyl]amino}méthyl)phénol ;
- 2-({méthyl[2-(4-nitrophényl)éthyl]amino}méthyl)-1,4-benzènediol ;
- acétate de 4-(diméthylamino)-2-méthoxy-6-({méthyl[2-(4-nitrophényl)éthyl]amino} méthyl)phényle ;
- 3,7-dihydroxy-*N*-{2-[4-({[3-(trifluorométhyl)phényl]sulfonyl}amino)phényl]éthyl}-2-naphthamide ;
- 3,7-dihydroxy-*N*-{3-[(4-méthyl-1-pipéridinyl)sulfonyl]benzyl}-2-naphthamide ;
ou un sel pharmaceutiquement acceptable d'un de ces derniers.

L'invention concerne en outre l'utilisation d'un composé de formule générale **(II)** telle que définie précédemment pour préparer un médicament destiné à traiter une maladie choisie parmi les maladies suivantes : les maladies prolifératives tumorales, et en particulier le cancer, les maladies prolifératives non tumorales, les maladies parasitaires, les infections virales, les maladies neurodégénératives, les myopathies, l'alopécie spontanée, l'alopécie induite par des produits exogènes et l'alopécie radio-induite.

De préférence, les composés de formule générale **(II)** seront utilisés pour préparer un médicament destiné à traiter une maladie choisie parmi les maladies suivantes : les maladies prolifératives tumorales, et en particulier le cancer, les maladies prolifératives non tumorales, les maladies parasitaires, les infections virales, l'alopécie spontanée, l'alopécie induite par des produits exogènes et l'alopécie radio-induite.

Tout particulièrement, les composés de formule générale **(II)** pourront être utilisés pour préparer un médicament destiné à traiter le cancer, et notamment la cancer du sein, les lymphomes, les cancers du cou et de la tête, le cancer du poumon, le cancer du colon, le cancer de la prostate et le cancer du pancréas.

L'invention concerne encore, à titre de produits industriels nouveaux, les composés de formule générale **(III)** 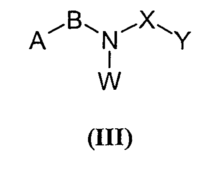 dans laquelle
A représente un radical **(A1)** 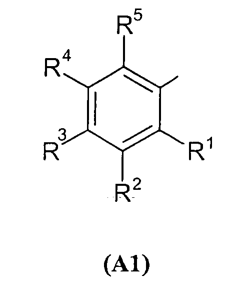 dans lequel deux des groupes R¹, R², R³, R⁴ et R⁵ représentent des atomes d'hydrogène et les trois autres sont choisis indépendamment parmi un atome d'hydrogène, un atome halogène et un radical alkyle, hydroxy, alkoxy, alkylcarbonyloxy, alkylthio ou NR⁶R⁷, étant entendu en outre que :
- ou bien R¹ et l'un de R² et R⁴ sont choisis indépendamment parmi un radical hydroxy, alkylcarbonyloxy et NR⁶R⁷,
- ou bien R² et l'un de R³ et R⁵ sont choisis indépendamment parmi un radical hydroxy, alkylcarbonyloxy et NR⁶R⁷,
- ou bien R⁴ et l'un de R³ et R⁵ sont choisis indépendamment parmi un radical hydroxy, alkylcarbonyloxy et NR⁶R⁷,
- ou encore l'un de R¹, R³ et R⁵ est choisi parmi un radical hydroxy, alkylcarbonyloxy et NR⁶R⁷, et le reste B-N(W)-X-Y est attaché au radical A par un atome d'azote,
R⁶ et R⁷ représentant, indépendamment à chaque fois qu'ils interviennent, un atome d'hydrogène ou un radical alkyle ou R⁶ et R⁷ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR⁸R⁹-, -O-, -S- et -NR¹⁰-, R⁸ et R⁹ représentant indépendamment à chaque fois qu'ils interviennent un atome d'hydrogène ou un radical alkyle, alkoxy, benzyloxycarbonylamino ou dialkylamino, et R¹⁰ représentant indépendamment à chaque fois qu'il intervient un atome d'hydrogène ou un radical alkyle,
ou encore A représente un radical **(A2)** 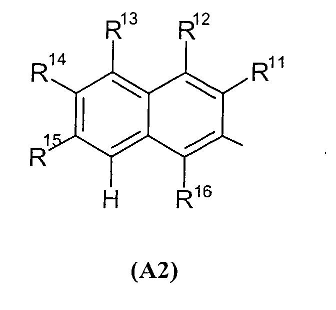 dans lequel :
- ou bien R¹¹ et l'un de R¹³, R¹⁴ et R¹⁵ représentent des radicaux hydroxy tandis que les autres radicaux parmi R¹³, R¹⁴ et R¹⁵ ainsi que R¹⁶ représentent des atomes d'hydrogène,
- ou bien R¹² et R¹⁶ représentent des radicaux hydroxy tandis que R¹¹, R¹³, R¹⁴ et R¹⁵ représentent des atomes d'hydrogène ;
B représente un radical -CO-, -NH-CO-(CH₂)ₙ- ou -(CH₂)ₚ-, n étant un entier de 0 à 3 et p étant un entier de 0 à 1 ;
W représente un atome d'hydrogène ou un radical alkyle ;
X représente un radical -(CH₂)_{q}-, -(CH₂)_{q}-NH- ou -CO-(CH₂)ᵣ-, q étant un entier de 1 à 6 et r un entier de 0 à 6 ;
ou encore l'ensemble B-N(W)-X-Y est tel qu'il représente le radical 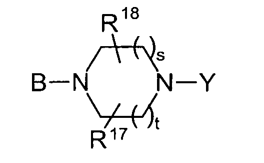 dans lequel B est tel que défini ci-dessus, t est un entier de 0 à 2, s est un entier de 0 à 1 et R¹⁷ et R¹⁸ représentent des radicaux choisis indépendamment parmi un atome d'hydrogène et un radical alkyle ;
et :
- lorsque X représente un radical -(CH₂)_{q}- ou -CO-(CH₂)ᵣ-, alors Y représente un radical 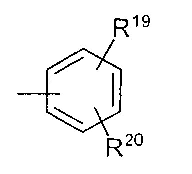 dans lequel R¹⁹ représente un radical -SO₂-NR²³R²⁴, -NH-SO₂-R²⁵ ou -O-P(O)(OR²⁶)(OR²⁷),
   R²³ et R²⁴ représentant indépendamment un atome d'hydrogène ou un radical alkyle, ou bien R²³ et R²⁴ représentant ensemble avec l'atome d'azote qui les porte un hétérocycle de 5 à 7 chaînons dont les chaînons complémentaires sont choisis indépendamment parmi -CHR²⁸-, -NR²⁹-, -O- et -S-, R²⁸ et R²⁹ représentant, indépendamment à chaque fois qu'ils interviennent, un atome d'hydrogène ou un radical alkyle,
   R²⁵ représentant un radical alkyle, haloalkyle ou l'un des radicaux aryle, hétéroaryle, aralkyle ou hétéroaralkyle dont le noyau aryle ou hétéroaryle est éventuellement substitué par un ou des radicaux choisis indépendamment parmi un atome halogène et des radicaux alkyle, haloalkyle, hydroxy, alkoxy ou nitro, sauf pour les éventuels atomes d'azote du noyau hétéroaryle pour lesquels les substituants éventuels sont choisis parmi des radicaux alkyle,
   R²⁶ et R²⁷ étant choisis indépendamment parmi des radicaux alkyle,
   et R²⁰ représente un atome d'hydrogène, un atome halogène ou un radical alkyle, alkoxy ou alkylthio ;
- lorsque X représente un radical -(CH₂)_{q}-NH- ou lorsque l'ensemble B-N(W)-X-Y est tel qu'il représente le radical 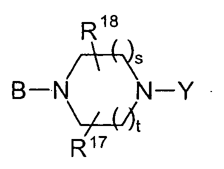 alors Y représente exclusivement un radical -SO₂-R³⁰ dans lequel R³⁰ représente un radical alkyle, haloalkyle ou l'un des radicaux aryle, hétéroaryle, aralkyle ou hétéroaralkyle dont le noyau aryle ou hétéroaryle est éventuellement substitué par un ou des radicaux choisis indépendamment parmi un atome halogène et des radicaux alkyle, haloalkyle, hydroxy, alkoxy ou nitro, sauf pour les éventuels atomes d'azote du noyau hétéroaryle pour lesquels les substituants éventuels sont choisis parmi des radicaux alkyle ;
   étant entendu en outre que lorsque l'ensemble B-N(W)-X-Y est tel qu'il représente le radical 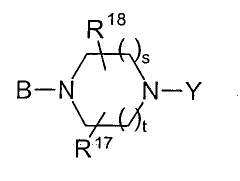 alors B représente exclusivement un radical -CO- ou -(CH₂)- ;
ou les sels de composés de formule générale **(III).**

En particulier, l'invention concerne, à titre de produits nouveaux, les composés de formule générale **(III)** suivants :
- 4-{2-[(3,7-dihydroxy-2-naphthoyl)amino]éthyl}phénylphosphate de diéthyle ;
- *N*-{2-[4-(aminosulfonyl)phényl)éthyl}-3,7-dihydroxy-2-naphthamide ;
- 3,7-dihydroxy-*N*-(2-{4-[(méthylsulfonyl)amino]phényl}éthyl)-2-naphthamide ;
- *N*-(2-{4-[(butylsulfonyl)amino]phényl}éthyl)-3,7-dihydroxy-2-naphthamide ;
- 3,7-dihydroxy-*N*-[2-(4-{[(4-méthylphényl)sulfonyl]amino}phényl)éthyl]-2-naphthamide ;
- 3,7-dihydroxy-*N*-(2-{4-[(1-naphthylsulfonyl)amino]phényl}éthyl)-2-naphthamide ;
- 3,7-dihydroxy-*N*-{2-[4-({[2-(trifluorométhyl)phényl]sulfonyl}amino)phényl]éthyl}-2-naphthamide ;
- *N*-(2-{4-[(benzylsulfonyl)amino]phényl}éthyl)-3,7-dihydroxy-2-naphthamide ;
- 3,7-dihydroxy-*N*-{2-[4-({[3-(trifluorométhyl)phényl]sulfonyl}amino)phényl]éthyl}-2-naphthamide ;
- 3,7-dihydroxy-*N*-[2-(4-{[(4-nitrophényl)sulfonyl]amino}phényl)éthyl]-2-naphthamide ;
- 3,7-dihydroxy-*N*-{2-[4-({[4-(trifluorométhyl)phényl]sulfonyl}amino) phényl]éthyl}-2-naphthamide ;
- 3,7-dihydroxy-*N*-(2-{4-[(thién-2-ylsulfonyl)amino]phényl}éthyl)-2-naphthamide ;
- 3,7-dihydroxy-*N*-[2-(4-{[(4-méthoxyphényl)sulfonyl]amino}phényl)éthyl]-2-naphthamide ;
- 3,7-dihydroxy-*N*-[2-(4-{[(1-méthyl-1H-imidazol-4-yl)sulfonyl]amino}phényl)éthyl]-2-naphthamide ;
- *N*-[2-(4-{[(4-fluorophényl)sulfonyl]amino}phényl)éthyl]-3,7-dihydroxy-2-naphthamide ;
- 3,7-dihydroxy-*N*-{3-[(4-méthyl-1-pipéridinyl)sulfonyl]benzyl}-2-naphthamide ;
- 3-({4-[(4-méthylphényl)sulfonyl]pipérazin-1-yl}carbonyl)naphthalène-2,6-diol ;
- 3-{[4-(méthylsulfonyl)pipérazin-1-yl]carbonyl}naphthalène-2,6-diol ;
- 3-{[4-(butylsulfonyl)pipérazin-1-yl]carbonyl}naphthalène-2,6-diol ;
et les sels de ces derniers.

L'invention concerne également, à titre de produits nouveaux, les composés de formule générale (I) suivants :
- 4-(diméthylamino)-2-({méthyl[2-(4-nitrophényl)éthyl]amino}méthyl)phénol ;
- 4-(4-aminophényl)-*N*-[4-(4-méthyl-1-pipérazinyl)phényl]butanamide ;
- 4-(diméthylamino)-2-méthoxy-6-({[2-(4-nitrophényl)éthyl]amino}méthyl)phénol ;
- 4-(diméthylamino)-2-({[2-(4-nitrophényl)éthyl]amino}méthyl)phénol ;
- 2-(diméthylamino)-6-méthoxy-4-({méthyl[2-(4-nitrophényl)éthyl]amino}méthyl) phénol ;
- 2-({méthyl[2-(4-nitrophényl)éthyl]amino}méthyl)-1,4-benzènediol ;
- acétate de 4-(diméthylamino)-2-méthoxy-6-({méthyl[2-(4-nitrophényl)éthyl]amino} méthyl)phényle ;
- 3,7-dihydroxy-*N*-[2-(4-nitrophényl)éthyl]-2-naphthamide ;
- *N*-[4-(diméthylamino)benzyl]-3,7-dihydroxy-2-naphthamide ;
et les sels de ces derniers.

L'invention concerne également, à titre de produit industriel nouveau répondant à la formule générale **(I),** le 5-(4-{[(1*E*)-amino(2-thiényl)méthylidène]amino}phényl)-N-[2-(diméthylamino) phényl]pentanamide.

L'invention concerne aussi des compositions pharmaceutiques comprenant, à titre de principe actif, un composé de formule générale **(III)** ou un sel pharmaceutiquement acceptable de ce dernier.

Elle a encore pour objet l'utilisation de composés de formule générale **(III)** ou de sels pharmaceutiquement acceptables de ces derniers pour préparer des médicaments destinés à traiter une maladie choisie parmi les maladies suivantes : les maladies prolifératives tumorales, et en particulier le cancer, les maladies prolifératives non tumorales, les maladies parasitaires, les infections virales, les maladies neurodégénératives, les myopathies, l'alopécie spontanée, l'alopécie induite par des produits exogènes et l'alopécie radio-induite.

De préférence, les composés de formule générale **(III)** seront utilisés pour préparer un médicament destiné à traiter une maladie choisie parmi les maladies suivantes : les maladies prolifératives tumorales, et en particulier le cancer, les maladies prolifératives non tumorales, les maladies parasitaires, les infections virales, l'alopécie spontanée, l'alopécie induite par des produits exogènes et l'alopécie radio-induite.

Tout particulièrement, les composés de formule générale **(III)** pourront être utilisés pour préparer un médicament destiné à traiter le cancer, et notamment la cancer du sein, les lymphomes, les cancers du cou et de la tête, le cancer du poumon, le cancer du colon, le cancer de la prostate et le cancer du pancréas.

D'une façon générale, les mêmes préférences que celles indiquées pour les composés de formule générale **(I)** sont par ailleurs applicables par analogie aux composés de formules générales **(II)** et **(III).**

L'invention concerne encore un procédé de préparation d'un composé de formule générale **(I).3** 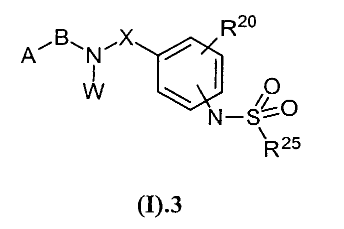 dans laquelle A, B, W, X, R²⁰ et R²⁵ ont la même signification que dans la formule générale **(I)**,
ledit procédé étant caractérisé en ce que l'on fait réagir le composé de formule générale **(I).2** 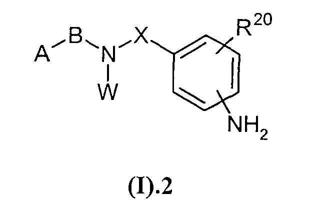 avec un composé de formule générale R²⁵-SO₂Cl dans un solvant aprotique (comme le tétrahydrofuranne, le dichlorométhane ou le diméthylformamide) et en présence d'une base (comme la pyridine, la triéthylamine ou une base supportée, par exemple la résine morpholinométhyl-polystyrène ; la base pouvant le cas échéant aussi servir de solvant réactionnel).

L'invention concerne encore un procédé de préparation d'un composé de formule générale **(I).7** 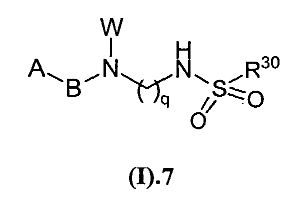 dans laquelle A, B, W, q et R³⁰ ont la même signification que dans la formule générale **(I)**,
ledit procédé étant caractérisé en ce que l'on fait réagir le composé de formule générale **(XXVII)** 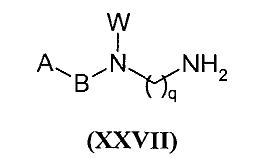 avec un composé de formule générale

R³⁰-SO₂Cl (XXVIII)

dans un solvant aprotique (comme le tétrahydrofuranne, le dichlorométhane ou le diméthylformamide) et en présence d'une base (comme la pyridine, la triéthylamine ou une base supportée, par exemple la résine morpholinométhyl-polystyrène ; la base pouvant le cas échéant aussi servir de solvant réactionnel).

L'invention concerne encore un procédé de préparation d'un composé de formule générale **(I).8** 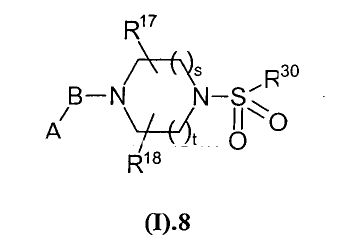 dans laquelle A, B, R¹⁷, R¹⁸, s, t et R³⁰ ont la même signification que dans la formule générale **(I)**,
ledit procédé étant caractérisé en ce que l'on fait réagir le composé de formule générale **(XXVII)*****bis*** 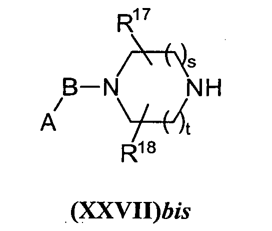 avec un composé de formule générale

R³⁰-SO₂Cl (XXVIII)

dans un solvant aprotique (comme le tétrahydrofuranne, le dichlorométhane ou .le diméthylformamide) et en présence d'une base (comme la pyridine, la triéthylamine ou une base supportée, par exemple la résine morpholinométhyl-polystyrène ; la base pouvant le cas échéant aussi servir de solvant réactionnel).

L'invention concerne encore un procédé de préparation d'un composé de formule générale **(I).9** 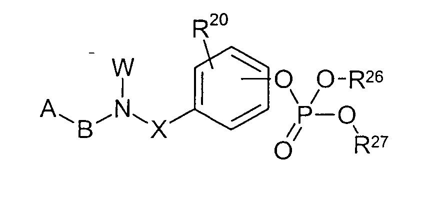 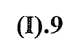 dans laquelle A, W, X, R²⁰, R²⁶ et R²⁷ ont la même signification que dans la formule générale **(I)**, et B représente le radical -CO- ou -CH₂-,
ledit procédé étant caractérisé en ce que l'on fait réagir l'amine de formule générale **(IV)**_{**p**} 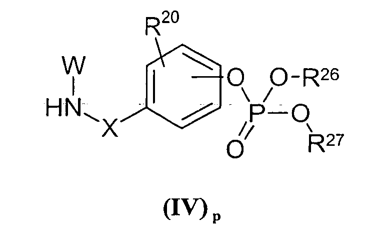 avec :
- soit, lorsque B représente le radical -CO-, avec un acide de formule générale

   A-CO₂H (V)

   dans un solvant aprotique (comme le tétrahydrofuranne, le dichlorométhane ou le diméthylformamide) et en présence d'un agent de couplage peptidique (comme le dicyclohexylcarbodiimide (DCC), le 1,1'-carbonyldiimidazole (CDI) ou le chlorhydrate de 1-(3-diméthylaminoproyl)-3-éthylcarbodiimide) ;
- soit, lorsque B représente le radical -CH₂-, avec un aldéhyde de formule générale

   A-CHO (VI)

   dans un solvant alcoolique (comme, par exemple, le méthanol) et en présence d'un agent réducteur (tel que NaBH₄, NaBH₃CN ou encore une résine contenant des ions borohydrure).

La composition pharmaceutique peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Conformément à l'invention, on peut préparer les composés de formule générale (I) par exemple par les procédés décrits ci-dessous.

### Préparation des composés de l'invention

Les procédés de préparation ci-après sont donnés à titre illustratif et l'homme du métier pourra leur faire subir les variations qu'il juge utiles, aussi bien en ce qui concerne les réactifs que les conditions et techniques des réactions.

### A) Cas où Y représente un radical phényle substitué

### 1. Cas où Y représente un radical du type nitrophényle :

Les composés de formule générale **(I)** dans lesquels Y représente un radical du type nitrophényle (ci-après désignés par « composés de sous-formule générale **(I).1** ») peuvent être facilement préparés selon des procédures identiques ou analogues à celles décrites dans la demande de brevet PCT WO 00/17190.

### 2. Cas où Y représente un radical du type aminophényle, dialkylaminophényle ou, alkylsulfonylamino ou encore un radical de formule (T) :

Dans le cas où Y représente un radical du type aminophényle, dialkylaminophényle, alkylsulfonylamino ou encore le radical de formule **(T)**, les composés de formule générale **(I)** peuvent être préparés à partir des dérivés nitrophényle de sous-formule générale **(I).1** selon les procédures représentées dans le schéma 1 ci-après.

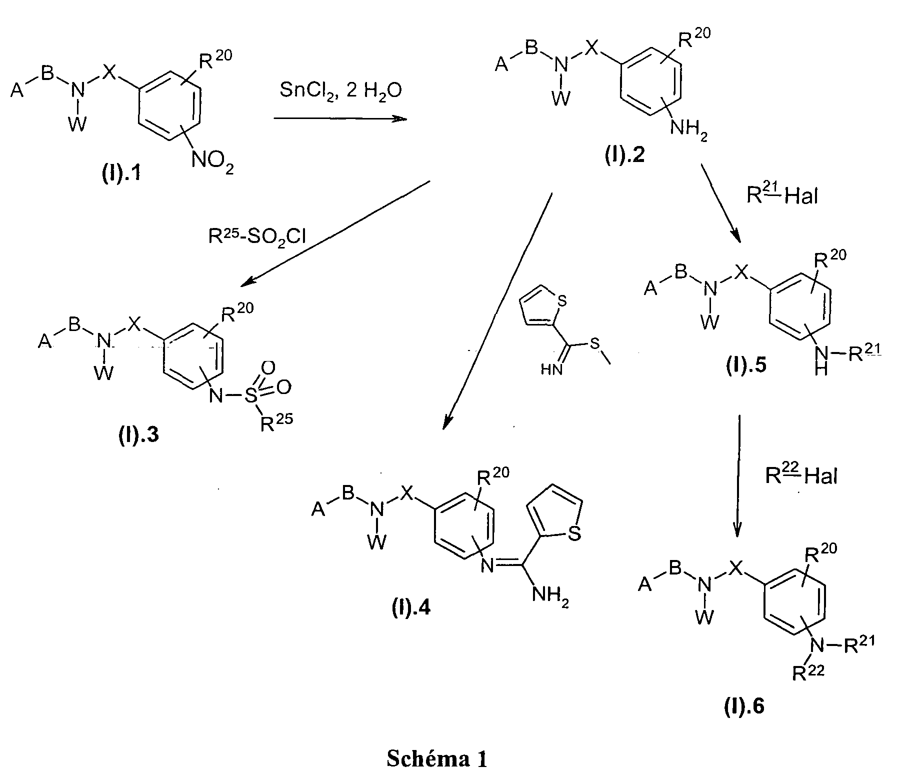

Dans le cas où aucun de X et B ne représente -CH₂-, les dérivés de type aminophényle de sous-formule générale **(I).2,** dans laquelle A, B, W, X et R²⁰ sont tels que définis ci-dessus peuvent être facilement obtenus, schéma 1, par réduction des composés de formule générale **(I).1,** par exemple, par action d'hydrogène en présence d'un catalyseur du type palladium sur charbon dans un solvant comme par exemple le méthanol, l'éthanol, le dichlorométhane ou le tétrahydrofuranne (THF). Dans le cas particulier où au moins l'un de X et B représente -CH₂-, la réduction de la fonction nitro peut être effectuée, par exemple, en chauffant le produit dans un solvant approprié tel que l'acétate d'éthyle avec un peu d'éthanol en présence de SnCl₂ (*J. Heterocyclic Chem*. (1987), **24**, 927-930 ; *Tetrahedron Letters* (1984), **25**(8), 839-842) ou en présence de SnCl₂ / Zn (*Synthesis.* (1996), **9**, 1076-1078), à l'aide de NaBH₄-BiCl₃ (*Synth. Comm.* (1995), **25**(23), 3799-3803) dans un solvant tel que l'éthanol, ou alors en utilisant du Ni Raney additionné d'hydrate d'hydrazine (*Monatshefte für Chemie*, (1995), **126**, 725-732), ou encore à l'aide d'indium dans un mélange d'éthanol et de chlorure d'ammonium à reflux (*Synlett* (1998), **9**, 1028).

Les composés de formule générale **(I)** dans lesquels Y représente le radical alkylsulfonylaminophényle (i.e. les composés de sous formule générale **(I).3)** peuvent être facilement préparés à partir des composés de formule générale **(I).2**, schéma 1, selon les méthodes classiques de synthèse des sulfonamides, par action d'un halogénure de sulfonyle sur un dérivé aminé, dans un solvant aprotique comme le THF, le dichlorométhane ou le diméthylformamide (DMF), en présence d'une base telle que la pyridine, la triéthylamine ou d'une base supportée telle que la résine morpholinométhyl-polystyrène ou encore en utilisant la pyridine comme solvant.

Les composés de formule générale **(I**) dans lesquels Y représente le radical **(T)** (i.e. les composés de sous formule générale **(I).4**) peuvent être facilement préparés à partir des composés de formule générale **(I).2,** schéma 1, selon des procédures identiques ou analogues à celles décrites dans les demandes de brevet PCT WO 00/17190.

Enfin, les dérivés du type alkylaminophényle ou dialkylaminophényle (respectivement les composés de sous-formules générales **(I).5** et **(I).6** représentés dans le schéma 1) peuvent être obtenus par mono- ou dialkylation des dérivés aminophényle de sous-formule générale **(I).2** selon des méthodes classiques connues de l'homme du métier. La mono-alkylation est réalisée par amination réductrice avec un aldéhyde ou par une substitution nucléophile par réaction avec un équivalent d'halogénoalkyle R²¹-Hal pour donner le dérivé monoalkylé de sous-formule générale **(I).5.** Une deuxième alkylation peut ensuite être réalisée le cas échéant au moyen d'un halogénoalkyle R²²-Hal pour donner le dérivé dialkylé de sous-formule générale **(I).6.**

Dans le cas particulier où R²¹ = R²² = -CH₃ et où aucun de X et B ne représente -CH₂-, le dérivé nitrophényle de sous-formule générale **(I).2** pourra être traité par des quantités adéquates de paraformaldéhyde sous atmosphère d'hydrogène dans un solvant comme l'éthanol et en présence d'un catalyseur du type palladium sur charbon pour donner le dérivé diméthylaminophényle de sous-formule générale **(I).6*****bis*** (cf. schéma 2 ci-dessous).

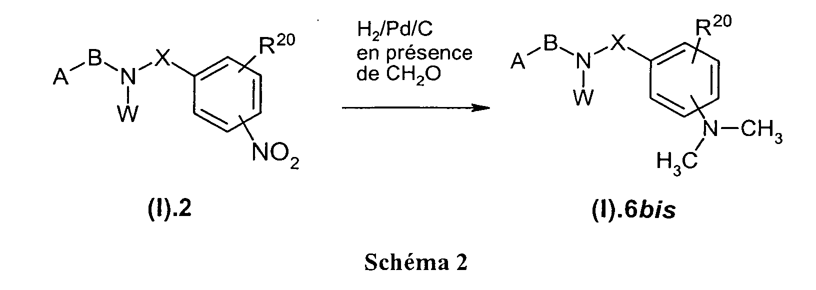

### 3. Autres cas où Y représente un radical du type phényle substitué :

Dans les autres cas non encore évoqués où Y représente un radical du type phényle substitué, la préparation des composés de formule générale **(I)** sera effectuée de façon classique pour l'homme du métier.

Lorsque les composés de formule générale **(I)** comprendront une fonction carboxamide (B = -CO-), ils pourront, par exemple, être préparés selon des méthodes de synthèse peptidique représentées dans le schéma 3 ci-après.

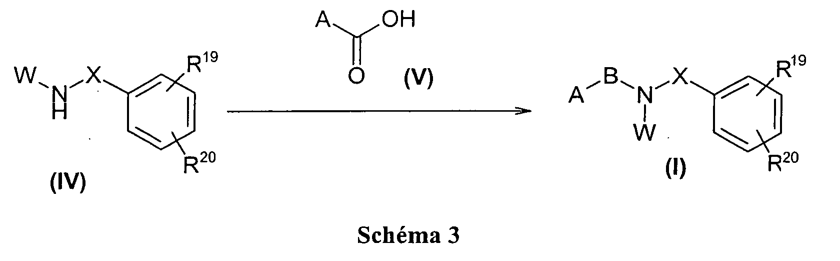

Les carboxamides de formule générale **(I)**, schéma 3, dans lesquels B représente -CO- et A, W, X, R¹⁹ et R²⁰ sont tels que définis précédemment, sont préparés par condensation des acides de formule générale **(V),** avec les amines de formule générale **(IV)** dans les conditions classiques de la synthèse peptidique (M. Bodanszky, The Practice of Peptide Synthesis, 145 (Springer-Verlag, 1984)), par exemple dans le THF, le dichlorométhane ou le DMF en présence d'un réactif de couplage tels que le dicyclohexylcarbodiimide (DCC), le 1,1'-carbonyldiimidazole (CDI) (*J. Med. Chem.* (1992), **35** (23), 4464-4472) ou le chlorhydrate de 1-(3-diméthylaminoproyl)-3-éthylcarbodiimide (EDC ou WSCI) (John Jones, The chemical synthesis of peptides, 54 (Clarendon Press, Oxford, 1991)).

Lorsque les composés de formule générale **(I)** seront tels que B = -CH₂-, ils pourront, par exemple, être préparés selon des méthodes d'amination réductrice représentées dans le schéma 4 ci-après.

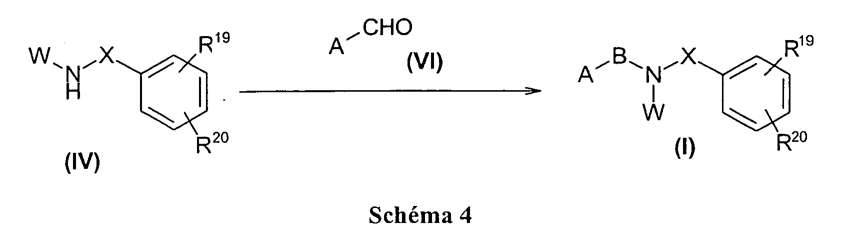

Les amines de formule générale **(I)**, schéma 4, dans lesquelles B représente -CH₂- et A, W, X, R₁₉ et R₂₀ sont tels que définis précédemment, sont préparées par réaction des aldéhydes de formule générale **(VI)** avec des amines de formule générale **(IV)** en milieu réducteur. La réaction a lieu dans un solvant alcoolique tel que, par exemple, le méthanol, et conduit à l'imine qui est ensuite transformée en amine par un agent réducteur tel que NaBH₄ ou NaBH₃CN ou encore une résine borohydrure Amberlite® IRA-400 (Aldrich ; 2,5 mmol BH₄⁻/ g de résine).

Lorsque les composés de formule générale **(I)** seront tels que B = -NH-CO-(CH₂)ₙ-, ils pourront, par exemple, être préparés selon les méthodes de synthèse représentées dans le schéma 5 ci-après.

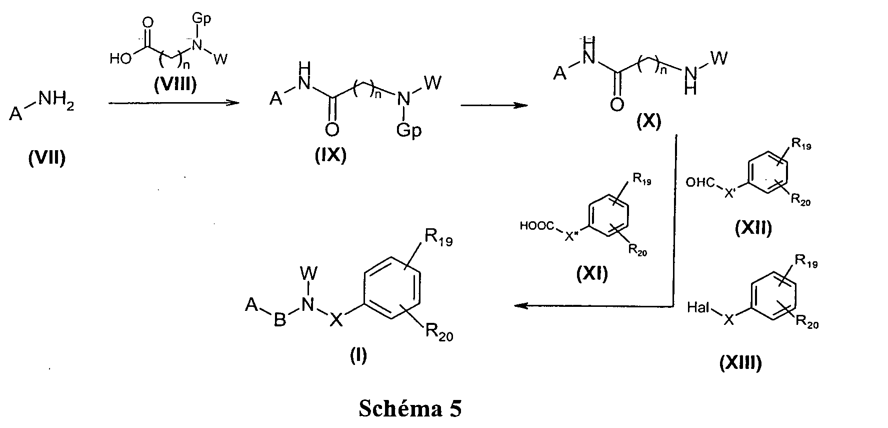

Les composés de formule générale **(I)**, schéma 5, dans lesquels B représente -NH-CO-(CH₂)ₙ- et A, W, X, R¹⁹, R²⁰ et n sont tels que définis précédemment, sont préparés par réaction des amines de formule générale **(X)** avec des acides carboxyliques de formule générale **(XI)** (dans laquelle X" est tel que X"-CO = X) selon des méthodes de synthèse peptidique décrites ci-dessus ou bien par réaction des mêmes amines de formule générale **(X)** avec des aldéhydes de formule générale **(XII)** (dans laquelle X' est tel que X'-CH₂ = X) dans les conditions d'amination réductrice décrites ci-dessus, ou encore par réaction des mêmes amines de formule générale **(X)** avec des dérivés halogénés de formule générale **(XIII)** selon des méthodes classiques connues de l'homme de métier. Les amines de formule générale **(X)** sont obtenues par condensation des amines de formule générale **(VII)** avec des acides de formule générale **(VIII)** dans lesquels A, W et n sont tels que définis précédemment et Gp représente un groupement protecteur de la fonction amine tel que, par exemple, un groupement carbamate, dans les conditions classiques de synthèse peptidique telles que décrites ci-dessus. La fonction amine est ensuite déprotégée (déprotection en milieu acide dans le cas où Gp représente un groupement carbamate tel que, par exemple, le groupement *tert*-butoxycarbonyle).

Lorsque les composés de formule générale **(I)** seront tels que B = -(CH₂)ₚ- avec p = 0 (autrement dit, B représente une liaison) et X = -CO-(CH₂)ᵣ-, ils pourront, par exemple, être préparés selon les méthodes de synthèse peptidique représentées dans le schéma 6 ci-après.

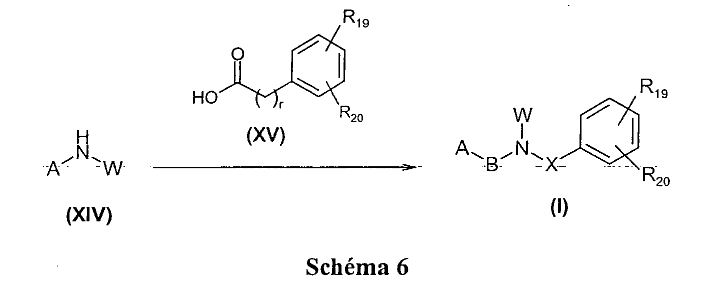

Les carboxamides de formule générale **(I),** schéma 6, dans lesquels B représente -(CH₂)ₚ- avec p = 0, X représente -CO-(CH₂)ᵣ- et A, W, R¹⁹, R²⁰ et r sont tels que définis précédemment, sont préparés par condensation des acides de formule générale **(XV),** avec les amines de formule générale **(XIV)** dans les conditions classiques de la synthèse peptidique décrites ci-dessus.

Lorsque les composés de formule générale **(I)** seront tels que B = -(CH₂)ₚ- avec p = 0 (autrement dit, B représente une liaison) et X = -(CH₂)_{q}-, ils pourront, par exemple, être préparés selon les méthodes de synthèse représentées dans le schéma 7 ci-après.

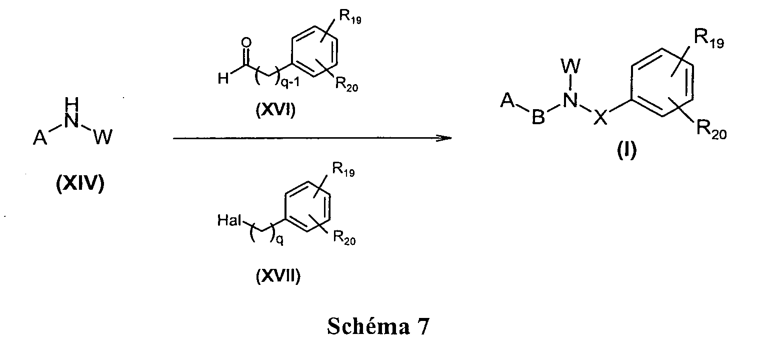

Les composés de formule générale **(I)**, schéma 7, dans lesquels B représente -(CH₂)ₚ- avec p = 0, X représente -(CH₂)_{q}-, A, W, R¹⁹, R²⁰ et q sont tels que définis précédemment, sont préparés par réaction des amines de formule générale **(XIV)** avec des aldéhydes de formule générale **(XVI)** dans les conditions d'amination réductrice décrites ci-dessus, ou bien par réaction des mêmes amines de formule générale **(XIV)** avec des dérivés halogénés de formule générale **(XVII)** selon des méthodes classiques connues de l'homme de métier.

### 4. Préparation des amines de formule générale (IV) :

Les amines non commerciales de formule générale **(IV)** dans lesquelles X représente -(CH₂)_{q}-, W représente H et R¹⁹ représente un radical -SO₂-NR²³R²⁴ (ci-après désignées par amines de formule générale **(IV)**_{**s**}**)**, peuvent notamment être obtenues en 6 étapes selon des méthodes de la littérature, et notamment selon la méthode représentée dans le schéma 8 ci-dessous.

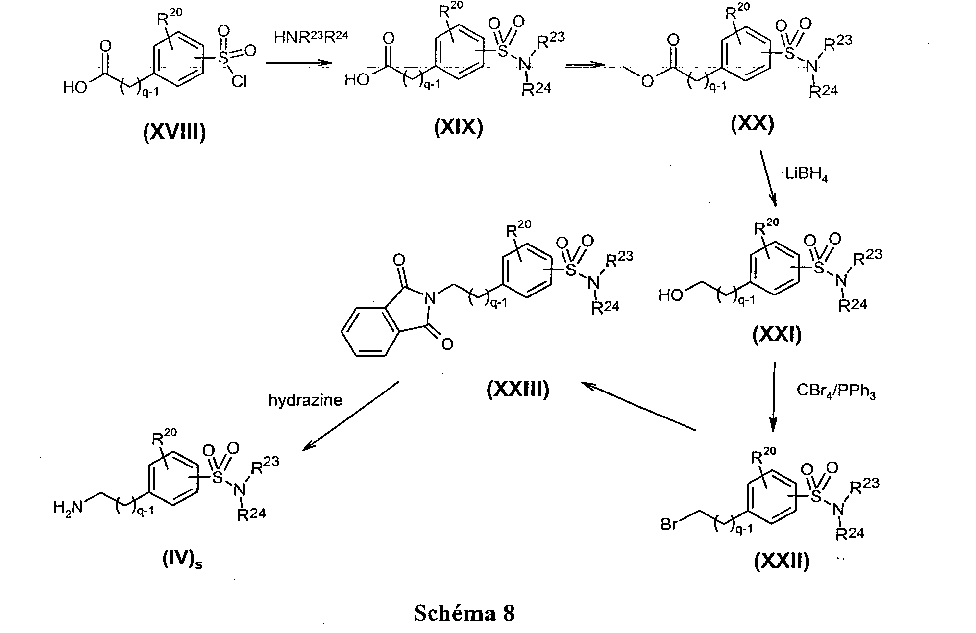

Par exemple, schéma 8, l'alcool de formule générale **(XXI)** est obtenu en 3 étapes à partir de l'acide de formule générale **(XVIII),** après passage au sulfonamide par action d'une amine primaire ou secondaire sur le chlorure de sulfonyle dans les conditions décrites précédemment pour la synthèse de sulfonamides, suivie d'une estérification, par exemple par un traitement avec du triméthylsilyldiazométhane dans un solvant alcoolique tel que, par exemple, le méthanol, et d'une réduction de la fonction ester par un agent réducteur tel que LiBH₄ dans un solvant aprotique polaire tel que, par exemple, le THF. La fonction alcool est ensuite halogénée par CBr₄ en présence de triphénylphosphine, puis convertie en phthalimide par traitement par du phthalimidate de potassium dans un solvant polaire tel que, par exemple, l'acétonitrile. Après coupure du phthalimide par addition d'hydrate d'hydrazine dans un solvant alcoolique tel que, par exemple, l'éthanol, l'amine de formule générale **(IV)**_{**s**} est obtenue.

Les amines de formule générale **(IV)** dans lesquelles R¹⁹ représente le groupe -O-P(O)(OR²⁶)(OR²⁷) (ci-après désignées par amines de formule générale **(IV)**_{**p**}**)** peuvent être obtenues en 2 étapes selon des méthodes de la littérature, et notamment, lorsque X représente un radical -(CH₂)_{q}-, selon la méthode représentée dans le schéma 9 ci-dessous.

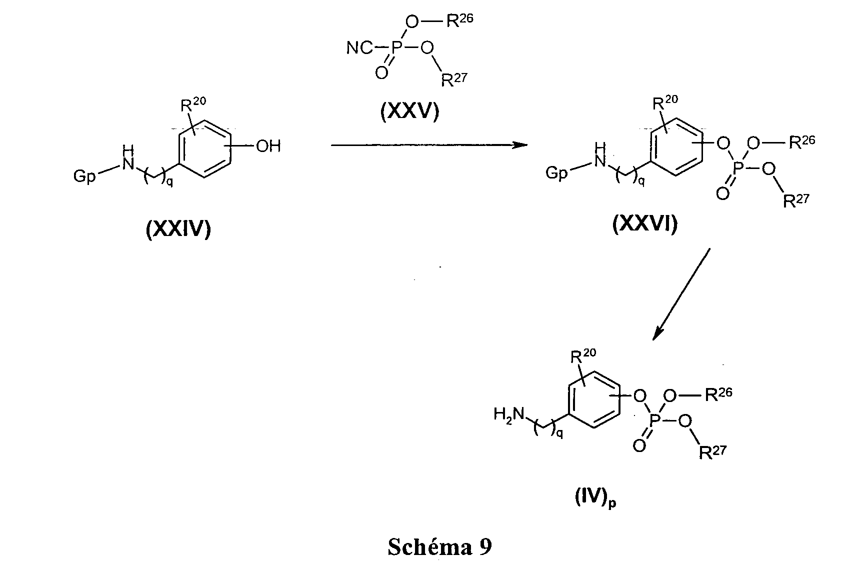

Selon ladite méthode, le phénol de formule générale **(XXIV)** est substitué, schéma 9, en utilisant un dérivé de type phosphonate, notamment le cyanophosphonate de formule générale **(XXV),** en présence d'une base telle que, par exemple, la triéthylamine dans un solvant tel que le dichlorométhane. Le groupement protecteur de la fonction amine (Gp) du composé de formule générale **(XXVI)** est ensuite coupé dans les conditions adéquates (par exemple en milieu acide dans le cas où Gp est un groupement de type carbamate, comme le groupement tert-butoxycarbonyle) pour donner finalement l'amine de formule générale **(IV)**ₚ.

Pour les autres amines de formule générale **(IV),** l'homme du métier pourra par exemple se référer à la demande de brevet PCT WO 00/17190.

### 5. Préparation de certains réactifs de départ :

Certains réactifs de départ ne sont pas commerciaux et devront être préparés selon des méthodes décrites dans la littérature. A titre d'exemple, la préparation des acides dihydroxy-2-naphtoïques (A = noyau naphtyle) peut être effectuée selon les méthodes décrites dans Marsilje et coll., *Bioorg. Med. Chem. Lett*. (2000), **10,** 477-481.

Les réactifs de départ dans lesquels A représente le radical phényle substitué par un groupement alkylcarbonyloxy sont obtenus à partir des phénols correspondants par action du chlorure d'acide correspondant en présence d'une base telle que, par exemple, la diisopropyléthylamine dans un solvant tel que le dichlorométhane.

La préparation de certains réactifs de départ nécessitera parfois l'emploi de réactions de protection et de déprotection bien connues de l'homme du métier qui pourra se référer, si besoin, à l'ouvrage suivant : T.W. Greene et P.G.M. Wuts, Protective Groups in Organic Synthesis, Second edition (Wiley-Interscience, 1991),

### B) Cas où Y représente un radical -SO₂R³⁰ :

Les composés de formule générale **(I)** dans lesquels Y représente le radical -SO₂-R³⁰ (i.e. les composés de sous-formule générale **(I).7** dans laquelle X représente le radical -(CH₂)_{q}-NH- défini précédemment et les composés de sous-formule générale **(I).8** dans laquelle l'ensemble -N(W)-X- représente un noyau pipérazinyle éventuellement substitué) peuvent être facilement préparés à partir des amines de formules générales **(XXVII)** et **(XXVII)*****bis*** (dans lesquelles A, B, W, R¹⁷, R¹⁸, q, s et t ont la même signification que dans la formule générale **(I))** selon des procédures de synthèse des sulfonamides telles que décrites plus haut (cf. schéma 10 ci-après).

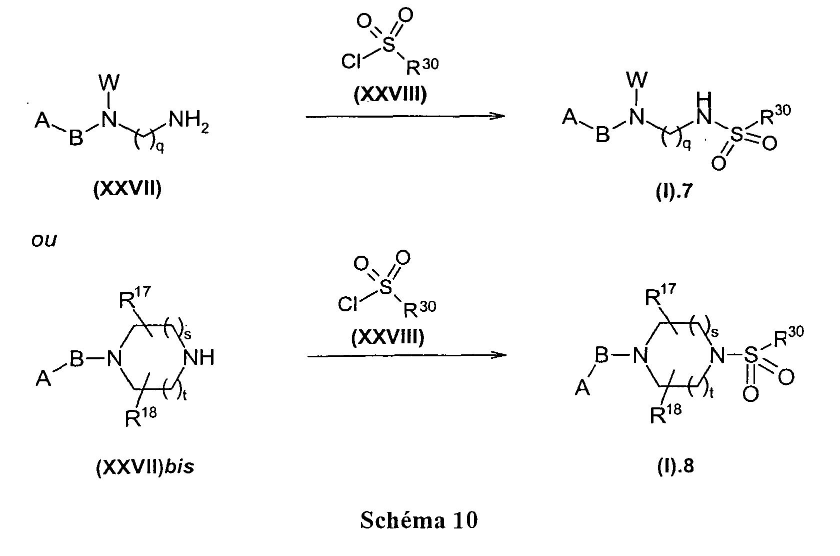

### Préparation des amines de formule générale (XXVII) et (XXVII)bis :

Lorsque B représente -CO- ou -(CH₂)ₚ- avec p = 1, les amines de formule générale **(XXVII)** et **(XXVII)*****bis*** pourront, par exemple, être préparées par les procédures représentées dans le schéma 11 ci-après.

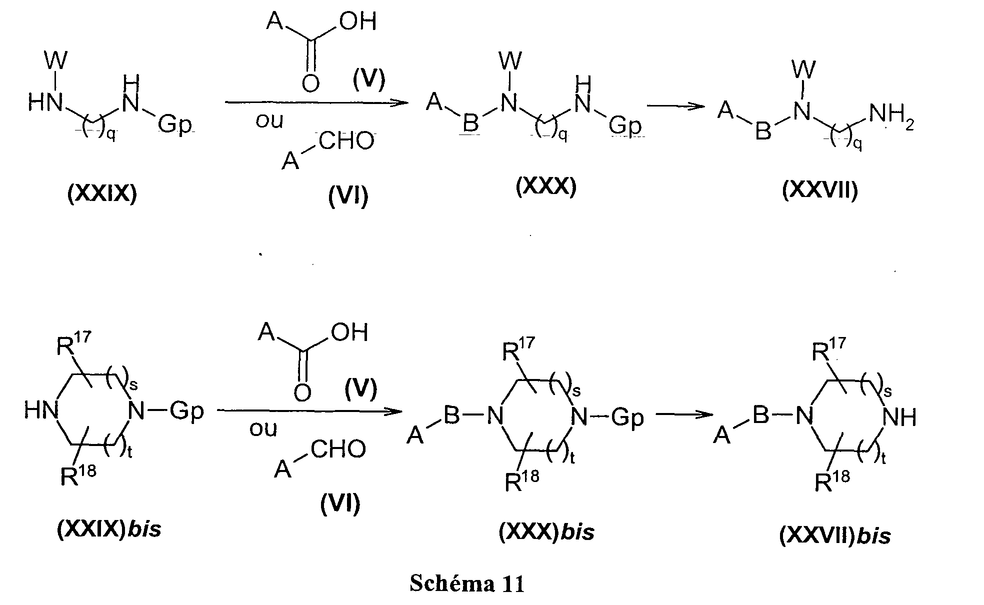

Les amines non commerciales de formule générale **(XXVII)** ou **(XXVII)*****bis*** dans lesquelles A, B, W, R¹⁷, R¹⁸, s, t et q sont tels que définis précédemment sont obtenues par les méthodes classiques par condensation d'acides carboxyliques de formule générale **(V)** et d'aldéhydes de formule générale **(VI)** sur des diamines linéaires de formule générale **(XXIX)** ou cycliques de formule générale **(XXIX)*****bis*****,** schéma 11, selon des méthodes analogues à celles décrites plus haut et dans lesquelles le groupement protecteur Gp de l'amine peut être un groupement carbamate tel que le groupement tert-butoxycarbonyle. La coupure du groupement protecteur est réalisée selon les méthodes connues, comme par exemple la coupure en milieu acide chlorhydrique pour le cas du groupement tert-butoxycarbonyle.

Lorsque les amines de formule générale **(XXVII)** seront telles que B = -(CH₂)ₚ- avec p = 0 (autrement dit, B représente une liaison), elles pourront, par exemple, être préparées selon les méthodes de synthèse représentées dans le schéma 12 ci-après.

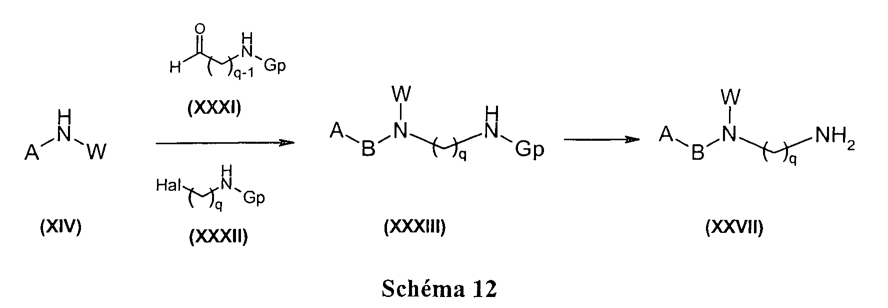

Les amines de formule générale **(XXVII)**, schéma 12, dans lesquelles B représente -(CH₂)ₚ- avec p = 0, X représente -(CH₂)_{q}-, A, W et q sont tels que définis précédemment et Gp est un groupe protecteur pour une fonction amine (par exemple un groupe protecteur de type carbamate comme le groupement tert-butoxycarbonyle), sont préparées par réaction des amines de formule générale **(XIV)** avec des aldéhydes de formule générale **(XXXI)** dans les conditions d'amination réductrice décrites ci-dessus, ou bien par réaction des mêmes amines de formule générale **(XIV)** avec des dérivés halogénés de formule générale **(XXXII)** selon des méthodes classiques connues de l'homme de métier, suivie par une étape de déprotection de l'intermédiaire de formule générale **(XXXIII)** effectuée dans des conditions classiques pour l'homme du métier.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### Exemples

### Caractérisation de certains composés par leur temps de rétention r.t.

Lorsqu'il est indiqué un temps de rétention r.t. pour les composés des exemples, celui-ci a été mesuré par Chromatographie Liquide Haute Performance associée à une spectrométrie de masse (*HPLC-MS*) en utilisant, selon ce qui est indiqué, les conditions d'élution suivantes :
- conditions I: passage d'un mélange acétonitrile-eau-acide trifluoroacétique 0-1000-0,2 (A) à un mélange acétonitrile-eau-acide trifluoroacétique 850-150-0,2 (B) par un gradient linéaire sur une période de 6 min puis élution avec le mélange B pur pendant 2 min.
- conditions II: passage d'un mélange acétonitrile-eau-acide trifluoroacétique 100-900-0,2 (A) à un mélange acétonitrile-eau-acide trifluoroacétique 850-150-0,2 (B) par un gradient linéaire sur une période de 6 min puis élution avec le mélange B pur pendant 2 min.
- conditions III : passage d'un mélange acétonitrile-eau-acide trifluoroacétique 50-950-0,2 (A) à un mélange acétonitrile-eau-acide trifluoroacétique 900-100-0,2 (B) par un gradient linéaire sur une période de 8,5 min puis élution avec le mélange B pur pendant 2 min.
- conditions IV : passage d'un mélange acétonitrile-eau-acide trifluoroacétique 50-950-0,2 (A) à un mélange acétonitrile-eau-acide trifluoroacétique 950-50-0 (B) par un gradient linéaire sur une période de 8,5 min puis élution avec le mélange B pur pendant 10,5 min.

### Exemple 1 : 4-(diméthylamino)-2-méthoxy-6-({méthyl[2-(4-nitrophényl)éthyl]-amino}méthyl)phénol

Il s'agit d'un intermédiaire de synthèse obtenu lors de la préparation du composé de l'exemple 80 de la demande WO 00/17190, le N'-(4-{2-[[5-(diméthylamino)-2-hydroxy-3-méthoxybenzyl]-(méthyl)amino]éthyl}-phényl)-2-thiophènecarboximidamide, selon la procédure décrite dans ce document.
Point de fusion : 91-93 °C.
MH+ = 360,30 ; t.r. = 3,40 min (conditions d'élution I).

### Exemple 2 : 4-(diméthylamino)-2-({méthyl[2-(4-nitrophényl)éthyl]amino}méthyl) phénol

0,5 g (3 mmol) de 5-(diméthylamino)-2-hydroxybenzaldéhyde (*Bull. Chem. Soc. Jpn.* (1978), 51 (8), 2433-2434) et 0,72 g (3,33 mmol) de chlorhydrate de N-méthyl-2-(4-nitrophényl)éthylamine sont mis en solution dans 30 ml de méthanol anhydre sous atmosphère inerte en présence de 0,65 ml de triéthylamine (4,5 mmol). Le mélange réactionnel est agité vigoureusement pendant 18 heures avant l'addition, par portions, de 126 mg (3,33 mmol) de NaBH₄. L'agitation est maintenue 4 heures supplémentaires avant addition de 10 ml d'eau glacée. Le mélange réactionnel est extrait par 2 fois 50 ml de CH₂Cl₂. La phase organique est lavée par 10 ml d'eau, séchée sur sulfate de sodium, filtrée et concentrée sous vide. Le résidu est purifié sur une colonne de silice (éluant : CH₂Cl₂/MeOH : 97/3). On obtient le produit attendu sous forme d'une huile marron avec un rendement de 34% (0,34 g).
RMN ¹H (DMSO d6, 200 MHz, δ) : 2,23 (s, 3H, CH₃) ; 2,71-2,77 (m, 8H, 2CH₃, CH₂) ; 2,96 (t, 3H, CH₂) ; 3,6 (s, 2H, CH₂) ; 6,48-6,53 (m, 3H aromatiques) ; 7,50-7,55 (m, 2H aromatiques) ; 8,13-8,17 (m, 2H aromatiques).
MH⁺ = 330,31 ; t.r. = 3,20 min (conditions d'élution I).

### Exemple 3 : chlorhydrate de 2,7-dihydroxy-N-{2-[4-[(2-thiényl(imino)méthyl) amino]phényl]éthyl}-2-naphtalènecarboxamide

[*il s'agit du composé de l'exemple 11 de la demande WO 00*/*17190*]

### Exemple 4: chlorhydrate de 3-[(3-{[amino(2-thiényl)méthylidène]amino}-benzyl)amino]-N-[4-(diméthylamino)phényl]propanamide

[*il s'agit du composé de l'exemple 50 de la demande WO 00*/*17190*]

### Exemple 5 : chlorhydrate de 4-(4-aminophényl)-N-[4-(4-méthyl-1-pipérazinyl)phényl]butanamide

### 5.1) 4-(4-nitrophényl)-N-[4-(4-méthyl-1-pipérazinyl)phényl]butanamide :

A une solution de 0,55 g (2,6 mmol) d'acide 4-(4-nitrophényl)butanoïque dans du dichlorométhane (30 ml) on ajoute de la 4-(N-méthylpipérazinyl)aniline (0,5 g ; 2,6 mmol), de l'hydroxybenzotriazole (0,39 g ; 2,86 mmol), du chlorhydrate de 1-(3-diméthylaminopropyl)-3-éthyl-carbodiimide (1,1 g; 5,7 mmol) et de la triéthylamine (0,8 ml ; 5,7 mmol). Le milieu réactionnel est agité pendant 16 heures à température ambiante, puis l'ensemble est dilué avec 15 ml d'eau et le produit est extrait par du dichlorométhane. La phase organique est séchée sur sulfate de sodium, filtrée et concentrée sous vide et le résidu est purifié sur colonne de silice (éluant : CH₂Cl₂/EtOH: 4/1). On obtient une huile claire avec un rendement de 84% (0,84 g).
RMN ¹H (CDCl₃, 100 MHz, δ) : 2,0-2,3 (m, 2H, CH₂) ; 2,3-2,5 (m, 5H, CH₂, CH₃) ; 2,5-2,7 (m, 4H, 2 CH₂) ; 2,7-3,0 (m, 2H, CH₂) ; 3,1-3,3 (m, 4H, 2CH₂) ; 6,9 (s, 1H, NH) ; 6,9-7,1 (m, 2H aromatiques); 7,3-7,5 (m, 4H aromatiques); 8,1-8,3 (m, 2H aromatiques).

### 5.2) Chlorhydrate de 4-(4-aminophényl)-N-[4-(4-méthyl-1-pipérazinyl)phényl]-butanamide :

A une solution de 0,84 g (2,2 mmol) d'intermédiaire 5.1 dans un mélange d'éthanol (150 ml) et de dichlorométhane (15 ml), on ajoute 0,1 g de palladium sur charbon (10%). L'ensemble est placé sous atmosphère d'hydrogène sous 1,5 bar de pression pendant 30 minutes. Le catalyseur est filtré et le solvant évaporé sous pression réduite. La base libre est obtenue avec un rendement de 71% (0,55 g ; 1,56 mmol) sous forme de solide blanc, puis mise en solution dans de l'éthanol glacé (45 ml) et additionnée de 4,6 ml (4,7 mmol) d'une solution 1*N* d'acide chlorhydrique dans l'éther. Après 30 minutes d'agitation à température ambiante, le solvant est évaporé à sec, puis le résidu est repris par de l'éther pour fournir le chlorhydrate attendu sous forme de solide marron clair avec un rendement de 97% (0,64 g). Point de fusion : 156-158 °C.
RMN ¹H (DMSO d6, 400 MHz, δ): 1,80-1,88 (m, 2H, CH₂); 2,27 (t, 2H, CH₂) ; 2,58 (t, 2H, CH₂) ; 2,78 (s, 3H, CH₃) ; 3,00-3,80 (m, 8H, 4 CH₂) ; 6,90-6,93 (m, 2H aromatiques) ; 7,04-7,06 (m, 2H aromatiques); 7,16-7,18 (m, 2H aromatiques) ; 7,47-7,49 (m, 2H aromatiques) ; 9,77 (s, 1H, NH).
MH⁺ = 353,23.

### Exemple 6 : 4-(diméthylamino)-2-méthoxy-6-({[2-(4-nitrophényl)éthyl]amino} méthyl)phénol

Le protocole expérimental utilisé est le même que celui décrit pour le composé de l'exemple 2, la 2-(4-nitrophényl)éthylamine remplaçant la N-méthyl-2-(4-nitrophényl)éthylamine et le 2-hydroxy-3-méthoxy-5-(diméthylamino)benzaldéhyde remplaçant le 2-hydroxy-5-(diméthylamino)benzaldéhyde. On obtient une huile marron.
RMN ¹H (CDCl₃, 400 MHz, δ): 2,85 (m, 6H, N(CH₃)₂) ; 2,97 (m, 4H, 2CH₂); 3,88 (s, 3H, OCH₃); 3,96 (s, 2H, CH₂); 6,06-6,07 (d, 1H aromatique); 6,36-6,37 (d, 1H aromatique); 7,36-7,37 (d, 2H aromatiques); 8,17-8,18 (d, 2H aromatiques).
MH+ = 346,20; t.r. = 3,40 min (conditions d'élution I).

### Exemple 7 : 4-(diméthylamino)-2-({[2-(4-nitrophényl)éthyl]amino}méthyl)phénol

Le protocole expérimental utilisé est le même que celui décrit pour le composé de l'exemple 2, la 2-(4-nitrophényl)éthylamine remplaçant la N-méthyl-2-(4-nitrophényl)éthylamine. On obtient une huile marron.
RMN ¹H (DMSO d6, 400 MHz, δ) : 2,71 (m, 6H, N(CH₃)₂) ; 2,75-2,89 (m, 4H, 2CH₂); 3,76 (s, 3H, CH₃) ; 6,55 (m, 3H, H aromatiques) ; 7,48-7,50 (d, 2H aromatiques) ; 8,12-8,14 (d, 2H aromatiques).
MH+ = 316,26 ; t.r. = 3,30 min (conditions d'élution I).

### Exemple 8 : 2-(diméthylamino)-6-méthoxy-4-({méthyl[2-(4-nitrophényl)éthyl] amino}méthyl)phénol

Le protocole expérimental utilisé est le même que celui décrit pour le composé de l'exemple 2, le 5-(diméthylamino)-4-hydroxy-3-methoxybenzaldéhyde, (*Bull. Chem. Soc. Jpn.* (1978), **51**(8), 2433-2434, remplaçant le 5-(diméthylamino)-3-méthoxy-2-hydroxybenzaldéhyde. On obtient une huile marron.
RMN ¹H (DMSO d6, 400 MHz, δ) : 2,61-2,64 (m, 2H, CH₂) ; 2,71 (m, 6H, N(CH₃)₂) ; 2,88-2,90 (m, 2H, CH₂) ; 3,66 (s, 2H, CH₂) ; 7,14-7,17 (m, 2H aromatiques); 7,48-7,51 (m, 2H aromatiques) ; 8,13-8,15 (m, 2H aromatiques).
MH+ = 360,27 ; t.r. = 3,30 min (conditions d'élution I).

### Exemple 9 : 2-({méthyl[2-(4-nitrophényl)éthyl]amino}méthyl)-1,4-benzènediol

Le protocole expérimental utilisé est le même que celui décrit pour le composé de l'exemple 2, le 2,5-dihydroxybenzaldéhyde remplaçant le 5-(diméthylamino)-3-méthoxy-2-hydroxybenzaldéhyde. On obtient une huile marron.
RMN ¹H (DMSO d6, 400 MHz, δ) : 2,22 (s, 3H, CH₃) ; 2,74 (t, 2H, CH₂) ; 2,95 (t, 2H, CH₂) ; 3,57 (s, 2H, CH₂) ; 6,48 (m, 3H aromatiques) ; 7,50-7,52 (d, 2H aromatiques) ; 8,13-8,15 (d, 2H aromatiques).
MH+ = 303,25 ; t.r. = 3,80 min (conditions d'élution I).

### Exemple 10 : acétate de 4-(diméthylamino)-2-méthoxy-6-({méthyl[2-(4-nitrophényl)éthyl]amino}méthyl)phényle

0,1 g (0,278 mmol) de 4-(diméthylamino)-2-méthoxy-6-({méthyl[2-(4-nitrophényl)éthyl]-amino}méthyl)phénol (composé de l'exemple 1) sont mis en solution dans 5 ml de dichlorométhane anhydre en présence de 49 µl (0,42 mmol; 1,5 éq.) de diisopropyléthylamine à 0 °C. 30 µl (0,42 mmol ; 1,5 éq.) de chlorure d'acétyle sont ajoutés goutte à goutte à la solution et l'ensemble est agité pendant 1 heure à température ambiante. Puis 10 ml de dichlorométhane sont ajoutés au milieu réactionnel qui est ensuite lavé par 3 fois 10 ml d'une solution saturée de chlorure de sodium. La phase organique est séchée sur sulfate de magnésium, filtrée et concentrée sous vide. Le résidu est purifié sur une colonne de silice (éluant : AcOEt/Heptane : 2/1) pour conduire à une huile jaune avec un rendement de 60%.
RMN ¹H (DMSO d6, 400 MHz, δ) : 2,16 (m, 6H, 2N(CH₃)₂) ; 2,59 (t, 2H, CH₂) ; 2,80 (m, 6H, 2 CH₃) ; 2,87 (t, 2H, CH₂) ; 3,31 (s, 2H, CH₂) ; 3,71 (s, 3H, OCH₃) ; 6,11 (s, 1H aromatique) ; 6,27 (s, 1H aromatique) ; 7,48 (m, 2H aromatiques); 8,11 (m, 2H aromatiques).
MH+ = 402,19 ; t.r. = 4,80 min (conditions d'élution I).

### Exemple 11 : 3,7-dihydroxy-N-[2-(4-nitrophényl)éthyl]-2-naphthamide

A une solution de 204 mg (1 mmol) d'acide 3,7-dihydroxy-2-naphtoïque dans du diméthylformamide (10 ml), on ajoute successivement 125 µl de 4-nitrophénéthylamine (1 mmol), 1 ml d'une solution 1*M* de N,N'-dicyclohexylcarbodiimide dans le dichlorométhane et 135 mg (1 mmol) de monohydrate d'hydroxybenzotriazole. La masse réactionnelle est agitée deux heures à température ambiante, diluée avec 100 ml d'eau et extraite deux fois avec 30 ml d'acétate d'éthyle. Les phases organiques sont rassemblées puis lavées successivement par 100 ml d'eau puis par 50 ml d'une solution saturée de chlorure de sodium. La phase organique est séchée sur sulfate de magnésium, filtrée et le solvant est éliminé par évaporation sous pression réduite. Le résidu est repris au dichlorométhane filtré et séché sous vide. On obtient 100 mg de produit sous la forme d'une poudre jaune (rendement de 30%). Point de fusion : > 250 °C.
MH+= 353,20 ; t.r. = 6,10 min (conditions d'élution I).

### Exemple 12 : N-[4-(diméthylamino)benzyl]-3,7-dihydroxy-2-naphthamide

Le protocole expérimental utilisé est le même que celui décrit pour le composé de l'exemple 11, la 4-diméthylaminobenzylamine remplaçant la 4-nitrophénéthylamine.
On obtient une huile jaune.
RMN-¹H (DMSO d6, 400 MHz, δ) : 2,86 (s, 6H); 4,42 (s, 2H); 6,70 (m, 2H) ; 7,04-7,20 (m, 5H); 7,58 (d, 1H); 8,29 (s, 1H); 9,32 (t, 1H) ; 9,55 (s, 1H) ; 11,78 (s, 1H).
MH+ = 337,20 ; t.r. = 4,20 min (conditions d'élution I).

### Exemple 13 : 4-{2-[(3,7-dihydroxy-2-naphthoyl)amino]éthyl}phénylphosphate de diéthyle

### 13.1) 2-{4-[(diéthoxyphosphoryl)oxy]phényl}éthylcarbamate de tert-butyle :

1,2 g (5 mmol) de N-Boc tyramine, 0,9 g (5,5 mmol) de diéthylcyanophosphonate et 1,4 ml (10 mmol) de triéthylamine sont mis en solution dans 5 ml de dichlorométhane à 0 °C sous atmosphère d'argon. Le mélange réactionnel est agité pendant 30 minutes à 0 °C, dilué avec 25 ml d'eau et extrait avec 2 fois 30 ml de dichlorométhane. Les phases organiques sont rassemblées puis lavées avec 50 ml d'eau puis 25 ml d'une solution saturée en chlorure de sodium. La phase organique est séchée sur sulfate de magnésium, filtrée et concentrée sous vide. On obtient le produit attendu sous forme d'une huile claire avec un rendement de 92% (1,73 g).

### 13.2) 4-(2-aminoéthyl)phénylphospate de diéthyle :

1,7 g (4,5 mmol) de l'intermédiaire 13.1 sont dissous dans 20 ml d'une solution 4M d'acide chlorhydrique dans du dioxane. Le mélange réactionnel est agité 1 heure à température ambiante puis concentré sous pression réduite. 20 ml d'une solution saturée d'hydrogénocarbonate de sodium sont ajoutés au mélange réactionnel qui est ensuite extrait par 2 fois 25 ml d'acétate d'éthyle. Le composé souhaité est obtenu avec un rendement de 62 % (0,76 g) et est utilisé dans l'étape suivante sans autre purification.

### 13.3) 4-{2-[(3,7-dihydroxy-2-naphthoyl)amino]éthyl}phénylphosphate de diéthyle :

Le protocole expérimental utilisé est le même que celui décrit pour le composé de l'exemple 11, l'intermédiaire 13.2 remplaçant la 4-nitrophénéthylamine. On obtient une poudre jaune. Point de fusion : 192-194°C.
MH+ = 460,20 ; t.r. = 9,60 min (conditions d'élution I).

### Exemple 14 : N-{2-[4-(aminosulfonyl)phényl]éthyl}-3,7-dihydroxy-2-naphthamide

Le protocole expérimental utilisé est le même que celui décrit pour le composé de l'exemple 11, le 4-(2-aminoéthyl)benzènesulfonamide remplaçant la 4-nitrophénéthylamine. On obtient une huile jaune.
RMN-¹H (DMSO d6, 400 MHz, δ) : 2,98 (t, 2H) ; 3,59 (q, 2H) ; 7,05-7,09 (m, 2H) ; 7,14 (s, 1H) ; 7,28 (s, 2H) ; 7,46 (d, 2H) ; 7,58 (d, 1H) ; 7,75 (d, 2H) ; 8,22 (s, 1H) ; 9,03 (t, 1H) ; 9,55 (s, 1H) ; 11,64 (s, 1H).
MH+ = 387,10 ; t.r. = 4,30 min (conditions d'élution I).

### Exemple 15 : 3,7-dihydroxy-N-[2-(4-aminophényl)éthyl]-2-naphthamide

Il s'agit d'un intermédiaire de synthèse obtenu lors de la préparation du composé de l'exemple 11 de la demande WO 00/17190, le 2,7-dihydroxy-N-{2-[4-[(2-thiényl (imino)méthyl)amino]phényl]éthyl}-2-napthalènecarboxamide, selon la procédure décrite dans ce document. On obtient un solide beige.
MH+ = 323,20 ; t.r. = 4,00 min (conditions d'élution I).

### Exemple 16 : 3,7-dihydroxy-N-(2-{4-[(méthylsulfonyl)amino]phényl}éthyl)-2-naphthamide

A une solution de 170 mg (0,5 mmol) du composé de l'exemple 15 dans de la pyridine (1 ml), on ajoute 44 µl (0,55 mmol) de chlorure de méthanesulfonyle. La masse réactionnelle est agitée 16 heures à température ambiante puis diluée avec 20 ml d'eau et extraite deux fois avec 30 ml d'acétate d'éthyle. Les phases organiques sont rassemblées puis lavées successivement avec 20 ml d'eau puis 20 ml d'une solution saturée de chlorure de sodium. La phase organique est séchée sur sulfate de magnésium, filtrée et le solvant est éliminé par évaporation sous pression réduite. Le résidu est élué sur silice avec un mélange d'acétate d'éthyle et de dichlorométhane (30/70). On obtient 65 mg de produit (rendement de 30%) sous la forme d'une poudre jaune. Point de fusion : 176-178 °C.
MH+ = 401,10 ; t.r. = 4,60 min (conditions d'élution II).

### Les composés des exemples 17 à 28 sont synthétisés selon la même stratégie que celle utilisée pour le composé de l'exemple 16.

### Exemple 17 : N-(2-{4-[(butylsulfonyl)amino]phényl}éthyl)-3,7-dihydroxy-2-naphthamide

Poudre jaune. Point de fusion : 193-195 °C.
MH+ = 443,20 ; t.r. = 5,50 min (conditions d'élution II).

### Exemple 18 : 3,7-dihydroxy-N-[2-(4-{[(4-méthylphényl)sulfonyl]amino}phényl) éthyl]-2-naphthamide

Poudre jaune. Point de fusion : 182-184 °C.
MH+ = 477,20 ; t.r. = 5,70 min (conditions d'élution II).

### Exemple 19 : 3,7-dihydroxy-N-(2-{4-[(1-naphthylsulfonyl)amino]phényl}éthyl)-2-naphthamide

Huile jaune.
MH+ = 513,20 ; t.r. = 9,70 min (conditions d'élution III).

### Exemple 20 : 3,7-dihydroxy-N-{2-[4-({[2-(trifluorométhyl)phényl]sulfonyl}amino) phényl]éthyl}-2-naphthamide

Huile jaune.
MH+ = 531,20 ; t.r. = 9,60 min (conditions d'élution III).

### Exemple 21 : N-(2-{4-[(benzylsulfonyl)amino]phényl}éthyl)-3,7-dihydroxy-2-naphthamide

Huile jaune.
MH+ = 477,20 ; t.r. = 9,20 min (conditions d'élution III).

### Exemple 22 : 3,7-dihydroxy-N-{2-[4-({[3-(trifluorométhyl)phényl]sulfonyl}amino) phényl]éthyl}-2-naphthamide

Huile marron.
MH+ = 531,20 ; t.r. = 9,80 min (conditions d'élution III).

### Exemple 23 : 3,7-dihydroxy-N-[2-(4-{[(4-nitrophényl)sulfonyl]amino}phényl) éthyl]-2-naphthamide

Huile orangée.
MH+ = 508,20 ; t.r. = 9,30 min (conditions d'élution III).

### Exemple 24 : 3,7-dihydroxy-N-{2-[4-({[4-(trifluorométhyl)phényl]sulfonyl}amino) phényl]éthyl}-2-naphthamide

Huile marron.
MH+ = 531,20 ; t.r. = 9,80 min (conditions d'élution III).

### Exemple 25 : 3,7-dihydroxy-N-(2-{4-[(thién-2-ylsulfonyl)amino]phényl}éthyl)-2-naphthamide

Huile jaune.
MH+ = 469,20 ; t.r. = 9,00 min (conditions d'élution III).

### Exemple 26 : 3,7-dihydroxy-N-[2-(4-{[(4-méthoxyphényl)sulfonyl]amino}phényl) éthyl]-2-naphthamide

Huile jaune.
MH+ = 493,20 ; t.r. = 9,10 min (conditions d'élution III).

### Exemple 27 : 3,7-dihydroxy-N-[2-(4-{[(1-méthyl-1H-imidazol-4-yl)sulfonyl]amino} phényl)éthyl]-2-naphthamide

Huile jaune.
MH+ = 467,20 ; t.r. = 7,90 min (conditions d'élution III).

### Exemple 28 : N-[2-(4-{[(4-fluorophényl)sulfonyl]amino}phényl)éthyl]-3,7-dihydroxy-2-naphthamide

Huile jaune.
MH+ = 481,20 ; t.r. = 9,20 min (conditions d'élution III).

### Exemple 29 : 3,7-dihydroxy-N-{3-[(4-méthyl-1-pipéridinyl)sulfonyl]benzyl}-2-naphthamide

### 29.1) 3-(4-méthylpipéridinosulfonyl)phénylméthanol :

11 g (50 mmol) d'acide 3-chlorosulfonylbenzoïque en suspension dans du dichlorométhane (300ml) sont traités avec 12,4 g (125 mmol) de 4-méthylpipéridine et le mélange réactionnel est maintenu sous agitation à température ambiante durant une nuit. La solution résultante est lavée successivement avec une solution aqueuse d'acide citrique à 10% et une solution saturée en chlorure de sodium, puis séchée (Na₂SO₄) et concentrée sous pression réduite pour donner un résidu visqueux incolore. Ce dernier est repris dans du méthanol et, en maintenant la température entre 0 °C et 10 °C, est titré par du triméthylsilyldiazométhane jusqu'à persistance d'une coloration jaune caractéristique. L'excès de réactif est détruit par quelques gouttes d'acide formique, et le milieux réactionnel est concentré sous pression réduite. Les traces d'acide et de méthanol sont éliminées par deux évaporations azéotropiques sous pression réduite avec du toluène. Le résidu, repris dans du tétrahydrofuranne (300 ml), est traité avec du borohydrure de lithium (2*N* dans du THF, 30 ml). Le milieu réactionnel est porté au reflux pendant 2 heures puis refroidi et hydrolysé avec une solution saturée en chlorure d'ammonium. Le mélange résultant est extrait à l'acétate d'éthyle puis la phase organique est lavée avec une solution saturée en chlorure de sodium, séchée (Na₂SO₄) et concentrée sous pression réduite. Le résidu est purifié sur une colonne de silice (éluant : 5% d'acétone dans du dichlorométhane) pour donner l'alcool benzylique attendu sous la forme d'un solide blanc.
RMN ¹H (DMSO d6, 400 MHz, δ): 0,84 (d, 3H) ; 1,13 (m, 2H) ; 1,26 (m, 1H) ; 1,63 (d, 2H) ; 2,17 (t, 2H) ; 3,59 (d, 2H) ; 4,59 (d, 2H) ; 5,42 (t, 1H) ; 7,60 (m, 3H) ; 7,67 (s, 1H).

### 29.2) 2-[3-(4-méthylpipéridinosulfonyl)benzyl]-1,3-isoindolinedione :

950 mg (3,5 mmol) d'alcool benzylique, 1,5 équivalent de triphénylphosphine et 1,5 équivalent de phtalimide en solution dans du dichlorométhane (50 ml) sont traités avec 1,5 équivalent de diazadicarboxylate de diisopropyle et le mélange réactionnel est agité la nuit durant à température ambiante, puis traité avec une solution saturée en chlorure d'ammonium, lavé avec une solution saturée en chlorure de sodium, séché (Na₂SO₄) et concentré sous pression réduite. Le résidu est purifié sur une colonne de silice (éluant : EtOAc/heptane 1/4) pour donner un solide blanc.
RMN ¹H (DMSO d6, 400 MHz, δ) : 0,79 (d, 3H) ; 1,05 (m, 2H) ; 1,26 (m, 1H) ; 1,57 (d, 2H) ; 2,18 (t, 2H) ; 3,56 (d, 2H) ; 4,89 (s, 2H) ; 7,6 (m, 3H) ; 7,68 (s, 1H) ; 7,88 (m, 4H).

### 29.3) 3-(4-méthylpipéridinosulfonyl)benzylamine :

L'intermédiaire 29.2 est traité par un excès d'hydrate d'hydrazine dans du méthanol et le mélange résultant est maintenu sous agitation à température ambiante durant une nuit, puis concentré sous pression réduite. Le résidu repris dans du dichlorométhane est lavé avec une solution saturée en chlorure de sodium. Après séchage et concentration, on obtient un solide blanc qui est purifié sur une colonne de silice (éluant : 10% MeOH/DCM) pour donner un solide blanc.
RMN ¹H (DMSO-d6, 400 MHz, δ) : 0,84 (d, 3H) ; 1,13 (m, 2H) ; 1,26 (m, 1H) ; 1,63 (d, 2H) ; 2,17 (t, 2H) ; 3,58 (d, 2H) ; 4,22 (d, 2H) ; 7,6 (m, 3H) ; 7,70 (s, 1H).

### 29.4) 3,7-dihydroxy-N-{3-[(4-méthyl-1-pipéridinyl)sulfonyl]benzyl}-2-naphthamide :

Le protocole expérimental utilisé est le même que celui décrit pour le composé de l'exemple 11, l'intermédiaire 29.3 remplaçant la 4-nitrophénéthylamine.
RMN ¹³C (DMSO-d6, 100 MHz, δ) : 21,4; 29,39; 32,90; 42,41 ; 46,19; 109,22; 111,06; 118,89; 121,59; 126,06; 126,14 ; 127,47 ; 127,57 ; 128,07 ; 129,58 ; 130,89 ; 132,15 ; 136,01 ; 140,98 ; 153,00 ; 153,67 ; 168,58.
MH+ = 455,20 ; t.r. = 9,90 min (conditions d'élution IV).

### Exemple 30 : chlorhydrate de 5-(4-{[(1E)-amino(2-thiényl)méthylidène] amino}phényl)-N-[2-(diméthylamino)phényl]pentanamide

### 30.1) N-[2-(diméthylamino)phényl]-5-(4-nitrophényl)pentanamide :

Le protocole expérimental utilisé est le même que celui décrit pour l'intermédiaire 5.1), la N,N-diméthyl-1,2-benzènediamine remplaçant la 4-(N-méthylpipérazinyl)aniline et l'acide 5-(4-nitrophény)pentanoïque remplaçant l'acide 4-(4-nitrophényl)butanoïque.

### 30.2) 5-(4-aminophényl)-N-[2-(diméthylamino)phényl]pentanamide :

Le protocole expérimental utilisé est le même que celui décrit pour l'intermédiaire 5.2, l'intermédiaire 30.1 remplaçant l'intermédiaire 5.1.

### 30.3) Chlorhydrate de 5-(4-{[(1E)-amino(2-thiényl)méthylidène]amino}phényl)-N-[2-(diméthylamino) phényl}pentanamide:

A une solution de 0,07 g (0,22 mmol) de l'intermédiaire 30.2 dans du 2-propanol (5 ml), on ajoute 0,071 g (0,24 mmol) d'iodhydrate de S-méthyl-2-thiophènethiocarboximide (*Ann. Chim.* (1962), **7,** 303-337). Après chauffage à 60 °C pendant 18 heures, le mélange réactionnel est concentré à sec sous pression réduite. Le résidu est repris dans de l'acétate d'éthyle et une solution saturée de carbonate de sodium. Après décantation, la phase organique est lavée successivement avec 2 fois 25 ml d'eau et 2 fois 25 ml d'une solution saturée en chlorure de sodium. La phase organique est séchée sur sulfate de magnésium, filtrée et évaporée sous pression réduite. Le résidu d'évaporation est purifié sur colonne de silice (éluant : acétate d'éthyle / heptane : 2/1). On obtient 0,06 g (rendement de 63%) de base libre. On prépare le chlorhydrate en dissolvant les 0,06 g (0,14 mmol) de base obtenue précédemment dans de l'acétone (10 ml) et en y ajoutant 0,43 ml d'une solution molaire d'acide chlorhydrique dans de l'éther diéthylique anhydre. Les cristaux obtenus sont filtrés et rincés à l'éther diéthylique pour donner, après séchage, 0,052 g du produit souhaité (rendement de 74%) sous forme d'un solide beige. Point de fusion : 145-148°C.
MH+ = 421,20 ; t.r. = 3,30 min (conditions d'élution II).

### Exemple 31 : 3-({4-[(4-méthylphényl)sulfonyl]pipérazin-1-yl}carbonyl) naphthalène-2,6-diol

### 31.1) 4-(3,7-dihydroxy-2-naphthoyl)pipérazine-1-carboxylate de tert-butyle :

Le protocole expérimental utilisé est le même que celui décrit pour le composé de l'exemple 11, la N-*tert*-butyloxycarbonylpipérazine remplaçant la 4-nitrophénéthylamine. On obtient un solide beige.

### 31.2) 3-(pipérazin-1-ylcarbonyl)naphthalène-2,6-diol :

A une solution de 1,9 g (5 mmol) de l'intermédiaire 31.1 dans de l'éthanol (20 ml), on ajoute 10 ml d'une solution 4*N* d'acide chlorhydrique dans du dioxane. La solution est agitée 2 heures à température ambiante, le solvant est évaporé et le résidu repris dans du dichlorométhane. Le précipité est filtré, lavé au dichlorométhane et séché sous vide. On obtient 1,35 g du produit attendu sous la forme d'une poudre blanche.

### 31.3) 3-({4-[(4-méthylphényl)sulfonyl]pipérazin-1-yl}carbonyl) naphthalène-2, 6-diol :

Le protocole expérimental utilisé est le même que celui décrit pour le composé de l'exemple 16, l'intermédiaire 31.2 remplaçant le composé de l'exemple 15 et le chlorure de para-toluènesulfonyle remplaçant le chlorure de méthanesulfonyle. On obtient une poudre jaune. Point de fusion : 135-137 °C.
MH+ = 427,20 ; t.r. = 8,30 min (conditions d'élution IV).

### Les composés des exemples 32 et 33 sont synthétisés selon la même stratégie que celle utilisée pour le composé de l'exemple 31.

### Exemple 32 : 3-{[4-(méthylsulfonyl)pipérazin-1-yl]carbonyl}naphthalène-2,6-diol

Poudre jaune. Point de fusion : 124-126 °C.
MH+ = 351,20 ; t.r. = 6,70 min (conditions d'élution IV).

### Exemple 33 : 3-{[4-(butylsulfonyl)pipérazin-1-yl]carbonyl}naphthalène-2,6-diol

Poudre jaune. Point de fusion : 105-110 °C.
MH+ = 393,20 ; t.r. = 7,90 min (conditions d'élution IV).

### Etude pharmacologique des composés de l'invention

### Protocoles des tests

### a) Mesure de l'activité phosphatase de l'enzyme recombinante Cdc25C purifiée

L'activité phosphatase de la protéine MBP-Cdc25C est évaluée par la déphosphorylation du 3-O-méthylfluorescéine-phosphate (OMFP) en 3-O-méthylfluorescéine (OMF) avec une détermination de la fluorescence à 475 nm du produit de la réaction. Cet essai permet d'identifier des inhibiteurs de l'enzyme recombinante cdc25. La préparation de la protéine de fusion MBP-cdc25C est décrite dans la demande de brevet PCT WO 01/44467.

La réaction est réalisée en format de plaque 384 puits sous un volume final de 50 µl. La protéine MBP-cdc25C (préparée comme décrit ci-dessus) est conservée dans le tampon d'élution suivant : 20 mM Tris-HCl pH 7,4 ; 250 mM NaCl ; 1mM EDTA ; 1 mM DTT ; 10 mM maltose. Elle est diluée à la concentration de 60 µM dans le tampon de réaction suivant : 50 mM Tris-HCl pH 8,2 ; 50 mM NaCl ; 1 mM DTT ; 20% glycérol. La mesure du bruit de fond est effectuée avec le tampon sans addition de l'enzyme. Les produits sont testés à des concentrations décroissantes à partir de 80 µM.. La réaction est initiée par l'ajout d'une solution OMFP à 500 µM finale (préparée extemporanément à partir d'une solution stock 12,5 mM dans du DMSO 100% (Sigma #M2629)) . Après 4 heures à 30 °C dans une plaque 384 puits à usage unique, la fluorescence mesurée à DO 475 nm est lue à l'aide d'un lecteur de plaque Victor2 (EGG-Wallac). La détermination de la concentration inhibant de 50% la réaction enzymatique est calculée à partir de trois expériences indépendantes. Seules les valeurs contenues dans la partie linéaire de la sigmoïde sont retenues pour l'analyse de régression linéaire.

### b) Mesure de l'état de phosphorylation de cdc2 :

Il s'agit de montrer que l'activité enzymatique de la phosphatase cdc25-C est inhibée in vivo en présence des inhibiteurs sélectionnés sur l'enzyme purifié. Quand cdc25-C est inhibée, la quantité de protéine cdc2 phosphorylée (inactive) augmente.

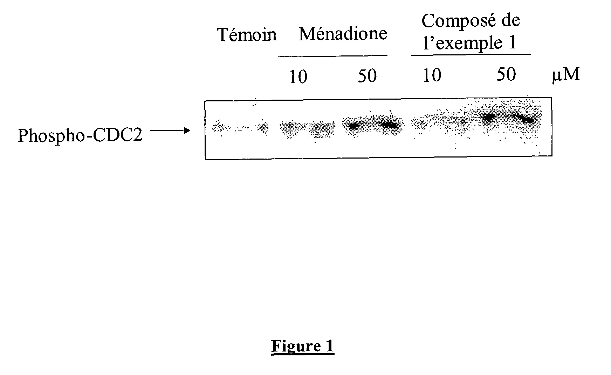

Les cellules de la lignée Mia PaCa2 sont ensemencées à raison de 450 000 cellules en boîte de Pétri 10 cm dans le milieu Eagle modifié de Dulbecco complété avec 10% de sérum de veau foetal. 48 heures après, les cellules sont traitées pendant 1 heure par le composé à tester ou la ménadione à 100 µM (inhibiteur de référence). Le milieu est renouvelé après lavage en PBS. 24 heures après les cellules sont grattées et lysées dans le tampon de lyse (Hépès 50 mM pH 7,5 ; NaCl 10 mM ; Triton X100 1% ; Glycérol 10% ; MgCl₂ 5 mM ; EDTA 1 mM ; Sodium Orthovanadate 1 mM ; protéase inhibiteur cocktail 1836170 Roche Diagnostics). Après centrifugation à 13 000 rpm pendant 10 minutes à 4°C, la concentration de protéines est déterminée dans le surnageant (Bio-Rad DC Protein assay kit) et ajustée à 10 µg. Le tampon de charge concentré 5 fois (TrisHCl 125 mM pH 7,4; SDS 10%; glycérol 50%; bleu de bromophénol 0,025% ; β-mercaptoéthanol 7%) est ajouté aux échantillons. Les échantillons sont chauffés pendant 10 minutes à 100 °C. Les échantillons sont déposés sous un volume de 40 µl sur des gels Tris/Glycine 12% (BioRad). La migration s'effectue pendant 1 heure à 180 V. Les protéines sont transférées sur une membrane de nitrocellulose (Hybond C, Amersham) dans des conditions semi-sèches. La membrane est bloquée pendant 1 heure dans du lait (BioRad) à 5% avec 0,1% de Tween 20. Puis elle est incubée pendant 6 heures avec l'anticorps primaire dirigé contre cdc2 phosphorylée (PhosphoPlus cdc2, 91115 New England BioLabs) dilué au 1/1300 ^{ième}. Après lavage en PBS-Tween 20 à 0,1%, la membrane est incubée pendant 1 h 30 avec l'anticorps secondaire anti-Immunoglobuline G de lapin (anti-rabbit IgG-HRP, sc2030, Santa Cruz) dilué au 1/40 000^{ième}. Les protéines sont révélées par électrochimioluminescence (western blotting detection system ECL⁺, Amersham) qui est détectée à l'aide de films photographiques (BioMax light, Sigma). Les images sont scannées (BioProfil scanner, Vilbert Lourmat) et traitées sous Powerpoint®. Le résultat obtenu est reproduit en figure 1 qui représente l'effet comparé de la ménadione et du composé de l'exemple 1 sur la phosphorylation de cdc2 dans la lignée Mia PaCa-2 (traitement de 3 heures par la ménadione ou le composé de l'exemple 1 et prélèvement 24 heures après).

### c) Caractérisation de l'activité anti-proliférative :

A titre d'exemple, on étudiera l'effet d'un traitement sur deux lignées de cellules humaines Mia-Paca2 et DU145 par les composés des exemples 1 à 5 décrits précédemment. Les lignées cellulaires DU145 (cellules humaines de cancer de la prostate) et Mia-PaCa2 (cellules humaines de cancer du pancréas) ont été acquises auprès de American Tissue Culture Collection (Rockville, Maryland, USA). Les cellules placées dans 80 µl de milieu Eagle modifié de Dulbecco (Gibco-Brl, Cergy-Pontoise, France) complété avec 10% de sérum foetal de veau inactivé par chauffage (Gibco-Brl, Cergy-Pontoise, France), 50000 unités/l de pénicilline et 50 mg/l streptomycine (Gibco-Brl, Cergy-Pontoise, France), et 2 mM de glutamine (Gibco-Brl, Cergy-Pontoise, France) ont été ensemencées sur une plaque de 96 puits au jour 0. Les cellules ont été traitées au jour 1 pendant 96 heures avec des concentrations croissantes de chacun des composés à tester jusqu'à 50 µg/ml. A la fin de cette période, la quantification de la prolifération cellulaire est évaluée par test colorimétrique en se basant sur le clivage du sel de tétrazolium WST1 par les déhydrogénases mitochondriales dans les cellules viables conduisant à la formation de formazan (Boehringer Mannheim, Meylan, France). Ces tests sont effectués en double avec 8 déterminations par concentration testée. Pour chaque composé à tester, les valeurs incluses dans la partie linéaire de la sigmoïde ont été retenues pour une analyse en régression linéaire et utilisées pour estimer la concentration inhibitrice CI₅₀. Les produits sont solubilisés dans le diméthylsulfoxide (DMSO) à 10⁻² M et utilisés en culture avec 0,5% DMSO en final.

### Résultats des tests

a) Les composés des exemples 1 à 11, 13 à 18 et 27 à 33 présentent une CI₅₀ inférieure ou égale à 100 µM sur l'activité phosphatase de l'enzyme recombinante cdc25-C purifiée.
b) L'activité inhibitrice du composé de l'exemple 1 sur la phosphatase cdc25C endogène des cellules humaines est montrée par l'augmentation croissante de la forme phosphorylée de cdc2 dans les cellules Mia-Paca2 traitées par des concentrations croissantes de ce composé. Cette augmentation est comparable à celle induite par la ménadione (voir figure 1).
c) Les composés des exemples 1 à 4, 6 à 11, 13, 14, 16 à 18, 22 et 28 à 31 présentent une CI₅₀ inférieure ou égale à 100 µM sur la prolifération cellulaire des lignées Mia-Paca2.
d) Les composés des exemples 1 à 4, 8 à 10, 13, 14, 16 à 18, 22 et 28 à 30 présentent une CI₅₀ inférieure ou égale à 100 µM sur la prolifération cellulaire des lignées DU-145.

## Revendications

1. Composé de formule générale **(III)** 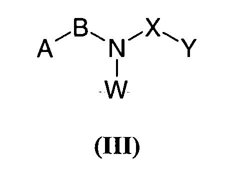 dans laquelle
A représente un radical **(A1)** 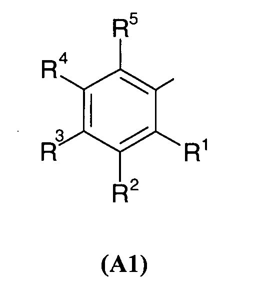 dans lequel deux des groupes R¹, R², R³, R⁴ et R⁵ représentent des atomes d'hydrogène et les trois autres sont choisis indépendamment parmi un atome d'hydrogène, un atome halogène et un radical alkyle, hydroxy, alkoxy, alkylcarbonyloxy, alkylthio ou NR⁶R⁷, étant entendu en outre que :
- ou bien R¹ et l'un de R² et R⁴ sont choisis indépendamment parmi un radical hydroxy, alkylcarbonyloxy et NR⁶R⁷,
- ou bien R² et l'un de R³ et R⁵ sont choisis indépendamment parmi un radical hydroxy, alkylcarbonyloxy et NR⁶R⁷,
- ou bien R⁴ et l'un de R³ et R⁵ sont choisis indépendamment parmi un radical hydroxy, alkylcarbonyloxy et NR⁶R⁷,
- ou encore l'un de R¹, R³ et R⁵ est choisi parmi un radical hydroxy, alkylcarbonyloxy et NR⁶R⁷, et le reste B-N(W)-X-Y est attaché au radical A par un atome d'azote,
R⁶ et R⁷ représentant, indépendamment à chaque fois qu'ils interviennent, un atome d'hydrogène ou un radical alkyle ou R⁶ et R⁷ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR⁸R⁹-, -O-, -S- et -NR¹⁰-, R⁸ et R⁹ représentant indépendamment à chaque fois qu'ils interviennent un atome d'hydrogène ou un radical alkyle, alkoxy, benzyloxycarbonylamino ou dialkylamino, et R¹⁰ représentant indépendamment à chaque fois qu'il intervient un atome d'hydrogène ou un radical alkyle,
ou encore A représente un radical **(A2)** 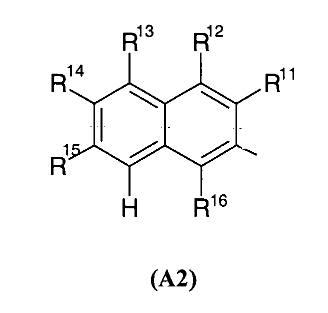 dans lequel :
- ou bien R¹¹ et l'un de R¹³, R¹⁴ et R¹⁵ représentent des radicaux hydroxy tandis que les autres radicaux parmi R¹³, R¹⁴ et R¹⁵ ainsi que R¹⁶ représentent des atomes d'hydrogène,
- ou bien R¹² et R¹⁶ représentent des radicaux hydroxy tandis que R¹¹, R¹³, R¹⁴ et R¹⁵ représentent des atomes d'hydrogène ;
B représente un radical -CO-, -NH-CO-(CH₂)ₙ- ou -(CH₂)ₚ-, n étant un entier de 0 à 3 et p étant un entier de 0 à 1 ;
W représente un atome d'hydrogène ou un radical alkyle ;
X représente un radical -(CH₂)_{q}-, -(CH₂)_{q}-NH- ou -CO-(CH₂)ᵣ-, q étant un entier de 1 à 6 et r un entier de 0 à 6 ;
et :
- lorsque X représente un radical -(CH₂)_{q}- ou -CO-(CH₂)ᵣ-, alors Y représente un radical 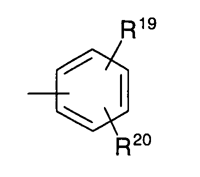 dans lequel R¹⁹ représente un radical -SO₂-NR²³R²⁴, -NH-SO₂-R²⁵ ou -O-P(O)(OR²⁶)(OR²⁷),
R²³ et R²⁴ représentant indépendamment un atome d'hydrogène ou un radical alkyle, ou bien R²³ et R²⁴ représentant ensemble avec l'atome d'azote qui les porte un hétérocycle de 5 à 7 chaînons dont les chaînons complémentaires sont choisis indépendamment parmi -CHR²⁸-, - NR²⁹-, -O- et -S-, R²⁸ et R²⁹ représentant, indépendamment à chaque fois qu'ils interviennent, un atome d'hydrogène ou un radical alkyle,
R²⁵ représentant un radical alkyle, haloalkyle ou l'un des radicaux aryle, hétéroaryle, aralkyle ou hétéroaralkyle dont le noyau aryle ou hétéroaryle est éventuellement substitué par un ou des radicaux choisis indépendamment parmi un atome halogène et des radicaux alkyle, haloalkyle, hydroxy, alkoxy ou nitro, sauf pour les éventuels atomes d'azote du noyau hétéroaryle pour lesquels les substituants éventuels sont choisis parmi des radicaux alkyle,
R²⁶ et R²⁷ étant choisis indépendamment parmi des radicaux alkyle,
et R²⁰ représente un atome d'hydrogène, un atome halogène ou un radical alkyle, alkoxy ou alkylthio ;
- lorsque X représente un radical -(CH₂)_{q}-NH- alors Y représente exclusivement un radical - SO₂-R³⁰ dans lequel R³⁰ représente un radical alkyle, haloalkyle ou l'un des radicaux aryle, hétéroaryle, aralkyle ou hétéroaralkyle dont le noyau aryle ou hétéroaryle est éventuellement substitué par un ou des radicaux choisis indépendamment parmi un atome halogène et des radicaux alkyle, haloalkyle, hydroxy, alkoxy ou nitro, sauf pour les éventuels atomes d'azote du noyau hétéroaryle pour lesquels les substituants éventuels sont choisis parmi des radicaux alkyle ;
ou un sel d'un composé de formule générale **(III)**.

2. Composé de formule générale **(III)** selon la revendication 1, **caractérisé en ce qu'**en outre :
• A représente un radical **(A1)** 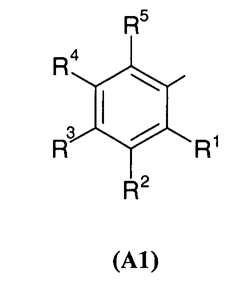 dans lequel deux des groupes R¹, R², R³, R⁴ et R⁵ représentent des atomes d'hydrogène et les trois autres sont choisis indépendamment parmi un atome d'hydrogène, un atome halogène et un radical alkyle, acétoxy, hydroxy, méthoxy ou NR⁶R⁷, étant entendu en outre que :
- ou bien R¹ et l'un de R² et R⁴ sont choisis indépendamment parmi un radical hydroxy, acétoxy et NR⁶R⁷,
- ou bien R² et l'un de R³ et R⁵ sont choisis indépendamment parmi un radical hydroxy, acétoxy et NR⁶R⁷,
- ou bien R⁴ et l'un de R³ et R⁵ sont choisis indépendamment parmi un radical hydroxy, acétoxy et NR⁶R⁷,
- ou encore l'un de R¹, R³ et R⁵ est choisi indépendamment parmi un radical hydroxy, acétoxy et NR⁶R⁷, et le reste B-N(W)-X-Y est attaché au radical A par un atome d'azote,
R⁶ et R⁷ représentant, indépendamment à chaque fois qu'ils interviennent, un atome d'hydrogène ou un radical alkyle comptant de 1 à 3 atomes de carbone ou R⁶ et R⁷ formant ensemble avec l'atome d'azote un hétérocycle comptant 6 chaînons et comportant de 1 à 2 hétéroatomes, les chaînons nécessaires pour compléter l'hétérocycle étant choisis indépendamment parmi les radicaux -CR⁸R⁹-, -O- et -NR¹⁰-, R⁸ et R⁹ représentant indépendamment à chaque fois qu'ils interviennent un atome d'hydrogène ou un radical alkyle, et R¹⁰ représentant indépendamment à chaque fois qu'il intervient un atome d'hydrogène ou un radical alkyle,
ou encore A représente un radical **(A2)** 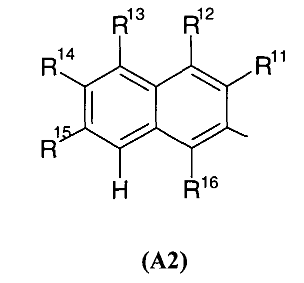 dans lequel R¹¹ et R¹⁵ représentent des radicaux hydroxy tandis que R¹², R¹³, R¹⁴ et R¹⁶ représentent des atomes d'hydrogène ;
• W représente un atome d'hydrogène ou un radical méthyle ; et
• X représentant un radical -(CH₂)_{q}-, -(CH₂)_{q}-NH- ou -CO-(CH₂)ᵣ, q étant un entier de 1 à 3 et r un entier de 0 à 4 ;
ou sel d'un tel composé.

3. Composé selon la revendication 1, **caractérisé en ce qu'**il s'agit de l'un des composés suivants :
- 4-{2-[(3,7-dihydroxy-2-naphthoyl)amino]éthyl}phénylphosphate de diéthyle ;
- *N*-{2-[4-(aminosulfonyl)phényl]éthyl}-3,7-dihydroxy-2-naphthamide ;
- 3,7-dihydroxy-*N*-(2-{4-[(méthylsulfonyl)amino]phényl}éthyl)-2-naphthamide ;
- *N*-(2-{4-[(butylsulfonyl)amino]phényl}éthyl)-3,7-dihydroxy-2-naphthamide ;
- 3,7-dihydroxy-*N*-[2-(4-{[(4-méthylphényl)sulfonyl]amino}phényl)éthyl]-2-naphthamide ;
- 3,7-dihydroxy-*N*-(2-{4-[(1-naphthylsulfonyl)amino]phényl}éthyl)-2-naphthamide ;
- 3,7-dihydroxy-*N*-{2-[4-({[2-(trifluorométhyl)phényl]sulfonyl}amino)phényl]éthyl}-2-naphthamide ;
- *N*-(2-{4-[(benzylsulfonyl)amino]phényl}éthyl)-3,7-dihydroxy-2-naphthamide ;
- 3,7-dihydroxy-*N*-{2-[4-({[3-(trifluorométhyl)phényl]sulfonyl}amino)phényl]éthyl}-2-naphthamide ;
- 3,7-dihydroxy-*N*-[2-(4-{[(4-nitrophényl)sulfonyl]amino}phényl)éthyl]-2-naphthamide ;
- 3,7-dihydroxy-*N*-{2-[4-({[4-(trifluorométhyl)phényl]sulfonyl}amino)phényl]éthyl}-2-naphthamide ;
- 3,7-dihydroxy-*N*-(2-{4-[(thién-2-ylsulfonyl)amino]phényl}éthyl)-2-naphthamide ;
- 3,7-dihydroxy-*N*-[2-(4-{[(4-méthoxyphényl)sulfonyl]amino}phényl)éthyl]-2-naphthamide ;
- 3,7-dihydroxy-*N*-[2-(4-{[(1-méthyl-1H-imidazol-4-yl)sulfonyl]amino}phényl)éthyl]-2-naphthamide ;
- *N*-[2-(4-{[(4-fluorophényl)sulfonyl]amino}phényl)éthyl]-3,7-dihydroxy-2-naphthamide ;
- 3,7-dihydroxy-*N*-{3-[(4-méthyl-1-pipéridinyl)sulfonyl]benzyl}-2-naphthamide ;
ou d'un sel d'un de ces derniers.

4. Procédé de préparation d'un composé de formule générale **(III).3** 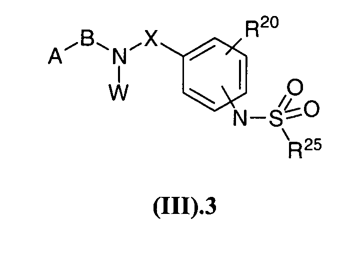 dans laquelle A, B, W, X, R²⁰ et R²⁵ ont la même signification que dans la formule générale **(III)** de la revendication 1,
ledit procédé étant **caractérisé en ce que** l'on fait réagir le composé de formule générale **(III).2** 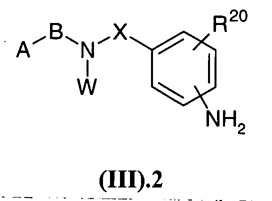 avec un composé de formule générale R²⁵-SO₂Cl dans un solvant aprotique et en présence d'une base.

5. Procédé de préparation d'un composé de formule générale **(III).7** 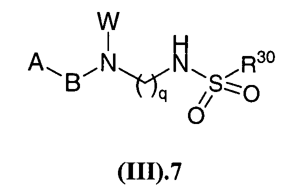 dans laquelle A, B, W, q et R³⁰ ont la même signification que dans la formule générale **(III)** de la revendication 1,
ledit procédé étant **caractérisé en ce que** l'on fait réagir le composé de formule générale **(XXIV)** 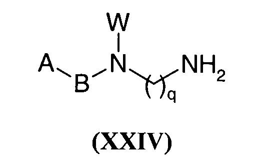 avec un composé de formule générale
R³⁰-SO₂Cl (XXV)
dans un solvant aprotique et en présence d'une base.

6. Procédé de préparation d'un composé de formule générale **(III).9** 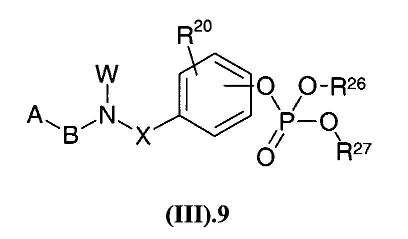 dans laquelle A, W, X, R²⁰, R²⁶ et R²⁷ ont la même signification que dans la formule générale **(III)** de la revendication 1, et B représente le radical -CO- ou -CH₂-,
ledit procédé étant **caractérisé en ce que** l'on fait réagir l'amine de formule générale **(IV)**_{**p**} 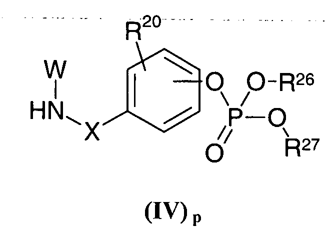 avec :
- soit, lorsque B représente le radical -CO-, un acide de formule générale
A-CO₂H (V)
dans un solvant aprotique et en présence d'un agent de couplage peptidique ;
- soit, lorsque B représente le radical -CH₂-, un aldéhyde de formule générale
A-CHO (VI)
dans un solvant alcoolique et en présence d'un agent réducteur.

7. A titre de médicament, un composé de formule générale **(III)** selon l'une des revendications 1 à 3 ou un sel pharmaceutiquement acceptable d'un tel composé.

8. Composition pharmaceutique comprenant, à titre de principe actif, un composé de formule générale **(III)** selon l'une des revendications 1 à 3 ou un sel pharmaceutiquement acceptable d'un tel composé.

9. Utilisation d'un composé de formule générale **(III)** selon la revendication 1, ou d'un sel pharmaceutiquement acceptable d'un tel composé, dans pour préparer des médicaments destinés à traiter une maladie choisie parmi les maladies suivantes : les maladies prolifératives tumorales, les maladies prolifératives non tumorales, les maladies parasitaires, les infections virales, les maladies neurodégénératives, les myopathies, l'alopécie spontanée, l'alopécie induite par des produits exogènes et l'alopécie radio-induite.

10. Utilisation selon la revendication 9, **caractérisée en ce que** la maladie destinée à être traitée est le cancer.

11. Utilisation selon la revendication 8, **caractérisée en ce que** le cancer est choisi parmi le cancer du sein, les lymphomes, les cancers du cou et de la tête, le cancer du poumon, le cancer du colon, le cancer de la prostate et le cancer du pancréas.
